(19) Europäisches Patentamt
European Patent Office
Office européen des brevets

(11) EP 1 743 895 A1

(12) **EUROPEAN PATENT APPLICATION**
published in accordance with Art. 158(3) EPC

(43) Date of publication:
**17.01.2007 Bulletin 2007/03**

(21) Application number: **05739090.8**

(22) Date of filing: **28.04.2005**

(51) Int Cl.:
***C07D 417/06*** (2006.01) ***A61K 31/554*** (2006.01)
***A61P 9/00*** (2006.01) ***A61P 9/04*** (2006.01)
***A61P 9/10*** (2006.01) ***A61P 9/12*** (2006.01)
***A61P 11/00*** (2006.01) ***A61P 21/02*** (2006.01)
***A61P 43/00*** (2006.01) ***C07D 417/14*** (2006.01)

(86) International application number:
**PCT/JP2005/008563**

(87) International publication number:
**WO 2005/105793 (10.11.2005 Gazette 2005/45)**

(84) Designated Contracting States:
**AT BE BG CH CY CZ DE DK EE ES FI FR GB GR HU IE IS IT LI LT LU MC NL PL PT RO SE SI SK TR**
Designated Extension States:
**AL BA HR LV MK YU**

(30) Priority: **28.04.2004 JP 2004134899**
**17.08.2004 JP 2004237533**
**12.04.2005 JP 2005141792**

(71) Applicant: **AETAS PHARMA CO., LTD.**
**Tokyo 103-0022 (JP)**

(72) Inventor: **KANEKO, Noboru**
**Oyama-shi, Tochigi 323-0022 (JP)**

(74) Representative: **Catherine, Alain et al**
**Cabinet Harlé & Phélip**
**7, rue de Madrid**
**75008 Paris (FR)**

(54) **MUSCLE RELAXATION ACCELERATOR AND THERAPEUTIC AGENT FOR MUSCULAR TISSUE DISEASES SUCH AS MUSCLE RELAXATION FAILURE**

(57) The present invention provides a drug serving as a muscular relaxation accelerating agent, a therapeutic agent for left ventricular diastolic dysfunction, a therapeutic agent for angina pectoris, a therapeutic agent for acute pulmonary edema, a drug for improving blood flow of microcirculatory system, a therapeutic and prophylactic agent for hypertension, a therapeutic and prophylactic agent for ventricular tachycardia and a therapeutic and prophylactic agent for torsade de pointes.

A muscular relaxation accelerating agent comprising 1,4-benzothiazepine derivatives represented by the following general formula [I] or a pharmaceutically acceptable salt thereof as an active ingredient;

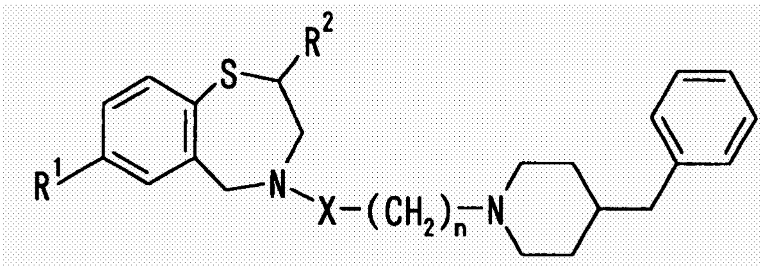

[I]

[wherein $R^1$ represents a hydrogen atom or C1-C3 lower alkoxy group; $R^2$ represents a hydrogen atom, C1-C3 lower alkoxy group or phenyl group (wherein the phenyl group may be substituted with 1 to 3 substituents selected from a group consisting of a hydroxyl group and a C1-C3 lower alkoxy group),

EP 1 743 895 A1

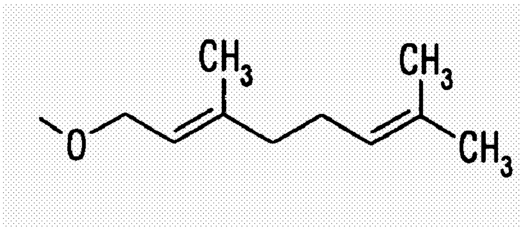 or 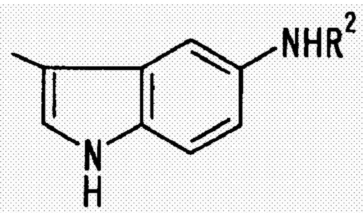

(wherein $R^3$ represents a C1-C3 acyl group); X represents -CO- or $-CH_2-$, and n represents an integer of 1 or 2.] Said muscular relaxation accelerating agent is the drug to make muscle relax to treat left ventricular diastolic dysfunction, angina pectoris and acute pulmonary edema, and improve blood flow of microcirculatory system to treat and prevent hypertension and ventricular tachycardia. Further, it is an effective drug for treatment and prevention for torsade de pointes.

FIG. 1

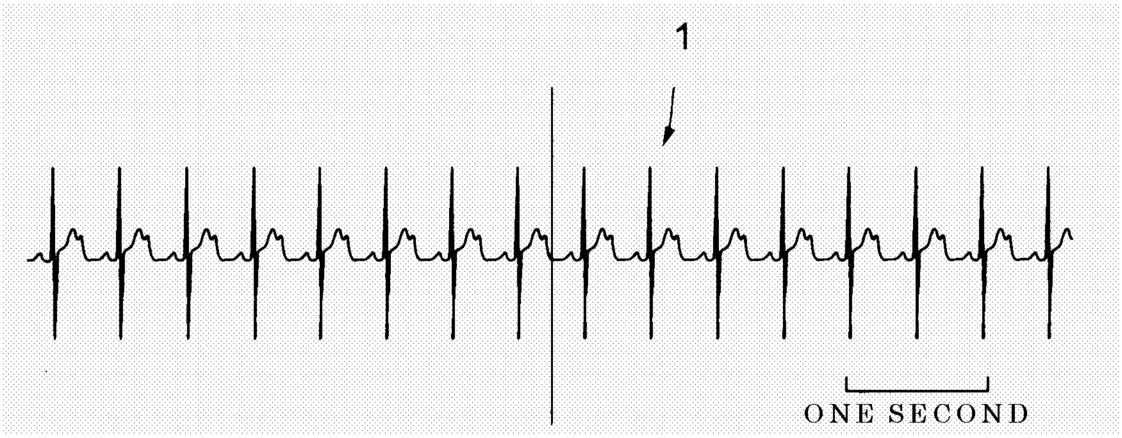

**Description**

TECHNICAL FIELD

The present invention relates to a compound with the function to accelerate relaxation of muscular tissues i.e., cardiac muscles, smooth muscles and skeletal muscles, and especially relaxation of myocardial tissues, and more particularly to a compound that accelerates relaxation of myocardial tissues and resolves failure of relaxation of myocardial tissues by administration to patients with insufficient myocardial relaxation; i.e., failure of myocardial relaxation.

Furthermore, this invention relates to a therapeutic agent or a prophylactic agent for diseases related to myocardial diastolic dysfunction, i.e., diseases associated with impaired myocardial relaxation, containing a compound that has the function to accelerate relaxation of myocardial tissues. Furthermore, this invention relates to a drug for improvement of microcirculation blood-flow containing a compound that has the function to accelerate relaxation of myocardial tissues, and particularly relates to a therapeutic agent or a prophylactic agent for cardiomegaly, subaortic stenosis (especially severe subaortic stenosis), aortic insufficiency, and angina pectoris, and more particularly relates to a therapeutic agent or a prophylactic agent for tachyarrhythmia such as polymorphic ventricular tachycardia, otherwise referred to as ventricular tachycardia or torsades de pointes. Furthermore, this invention relates to a therapeutic agent or a prophylactic agent for diseases associated with cardiomyopathy, such as left and right ventricular diastolic failure, left ventricular diastolic failure, acute/chronic pulmonary congestion, acute pulmonary edema, and left ventricular cardiomyopathy containing a compound that has the function to accelerate relaxation of myocardial tissues agent. Furthermore, this invention relates to a therapeutic agent or a prophylactic agent for heart failure containing a compound that has the function to facilitate relaxation of myocardial tissues. In addition, this invention relates to a therapeutic agent or a prophylactic agent for acute pulmonary edema containing a compound that has the function to accelerate muscular tissue relaxation. Moreover, this invention relates to a therapeutic agent or a prophylactic agent for angina pectoris, especially intramyocardial microvascular angina pectoris, containing a compound that has the function to facilitate muscular tissue relaxation. Moreover, this invention relates to a therapeutic agent for hypertension and a therapeutic agent for catecholamine hypertension containing a compound that has the function to accelerate muscle tissue relaxation. Furthermore, this invention relates to a therapeutic agent or a prophylactic agent for arrhythmia which develops when calcium overload occurs in myocardial cells, containing a compound that has the function to accelerate muscle tissue relaxation, and also relates to a therapeutic agent or a prophylactic agent for catecholamine-induced arrhythmia which develops when calcium overload occurs.

BACKGROUND ART

The pathological mechanism of heart failure is thought to be due to contractile failure of the myocardium, and drugs which enhance myocardium contraction are used as therapeutic agents for heart failure; for example, these include (1) digitalis (such as digoxin, digitoxin, and digilanogen C), (2) catecholamines (such as dopamine, dobutamine, and denopan), (3) phosphodiesterase inhibitors (such as PDE III inhibitors, amrinone, milrinone, and vesnarinone), and (4) calcium sensitizers (pimobendan). Of these drugs, those in categories (1) to (3) above accelerate myocardial contraction by increasing the intracellular concentration of calcium ions, and the calcium sensitizer in category (4) accelerates myocardial contraction by enhancing the calcium ion sensitivity of troponin C, which is contraction regulatory protein for myocardium.

As an alternative to the above drugs, a therapeutic agent or a prophylactic agent containing an active ingredient and having an inhibitory effect on leakage of calcium ions from the sarcoplasmic reticulum through improvement and/or stabilization of ryanodine receptor function, is suggested (See Japanese published unexamined application No. 2003-95977).

However, recently, it has been discovered that in case of heart failure, many patients develop heart failure regardless of retention of their left ventricular systolic function, and such patients account for 40% of patients with heart failure. In addition, the prognosis of these patients is not always good. Such patients with heart failure have no left ventricular dilation; hence, a function of left ventricle dilation becomes a cause of heart failure, which is referred to as diastolic heart failure.

Contraction and relaxation of muscle which has myocardium, skeletal muscles and smooth muscles are essential for the function of organs or tissues attached to the muscles. Substances which enhance muscular contraction have been studied for treatment of contraction and relaxation of these muscles, because significant energy is required for muscular contraction at the time of blood outflow. For example, in cardiac diseases, drugs that enhance muscular contraction serve as therapeutic agents for heart failure, as mentioned above, and in the case of diseases involving blood vessels, such drugs allow elevation of blood pressure by enhancing contraction of blood vessels. On the contrary, β-blockers are examples of drugs that decrease oxygen consumption of muscle tissues by depressing cardiac contractility, and such drugs are used as therapeutic agents for angina.

Torsades de pointes, which occurs more often in patients with long QT syndromes, is a subtype of polymorphic ventricular tachycardia, and an arrhythmia in which the QRS axis continuously changes and the QRS configuration periodically changes, with twisting centering on the baseline. Most cases of torsades de pointes resolve spontaneously, but are often repetitive, and this is of concern since the disease can be life threatening because syncope may occur or ventricular fibrillation may develop. Development of torsades de pointes can be caused by certain kinds of antiarrhythmic agents, antihistamines, and antipsychotics such as chlorpromazine, and is also induced by electrolyte abnormalities such as those that occur in hypomagnesemia and hypokalemia. In addition, it is well known that the disease is initiated by quinidine, disopyramide, procainamide, propafenone, and cibenzoline, which are classified as Class IA drugs in the Vaughan Williams classification of antiarrhythmic agents and are administered for treatment of arrhythmia, and by amiodarone and nifekalant hydrochloride, which are classified as Class III drugs in the Vaughan Williams classification. These antiarrhythmic agents prolong QT interval, and torsades de pointes can be experimentally induced by clofilium, which is also classified as a Class III drug in the Vaughan Williams classification.

A sharp distinction is made between heart failure due to systolic failure and that due to diastolic failure, because these conditions vary in pathogenesis. Therefore, therapeutic approaches to these diseases are different, and it has been suggested that therapeutic agents for acute exacerbation and therapeutic agents for the chronic period are not appropriate for treatment of left ventricular diastolic failure. All known drugs are not perfect; for example, β-blockers that have been used as therapeutic agents for heart failure are not adequate because these agents affect myocardial contraction; therefore, development of a magic bullet to improve myocardial relaxation, i.e., a therapeutic agent for heart failure caused by diastolic failure, is anticipated.

The object of this invention is to provide a therapeutic agent as a magic bullet for myocardial relaxation failure, which may account for 40% of cases of ordinary heart failure, i.e., diastolic heart failure caused by insufficiency of dilation.

Many diseases develop due to hypofunction of muscular relaxation, and these diseases can be improved by facilitation of muscular relaxation. However, a method that accelerates muscular relaxation without having effects on muscular contraction is required. For example, the heart acts as a pump through repeated contraction and relaxation; coronary perfusion is mainly performed at diastole, and acceleration of muscular relaxation results in improvement of coronary circulation. Hence, patients with cardiomegaly, and especially those with severe aortic stenosis or aortic regurgitation, develop angina pectoris if coronary circulation at diastole is disturbed, and a method to correct such impaired coronary circulation without having an effect on muscular contraction is required. Hypertensive heart disease, idiopathic hypertrophic cardiomyopathy, valvular disease of the heart, cardiac hypertrophy in the elderly and myocardial damage accompanied by age-related impaired myocardial relaxation that presents as ST segment depression on the electrocardiogram may also develop. Similarly, in these cases, a method to treat and prevent the above-mentioned diseases by facilitating myocardial relaxation without effects on muscular contraction is required. Catecholamines can be used as drugs that facilitate muscle tissue relaxation and can act as a therapeutic agent for hypertension, because expansion of peripheral blood vessels by smooth muscle relaxation relieves a rapid elevation in blood pressure and prevents a rapid blood pressure drop; however, a method to facilitate smooth muscle relaxation of the blood vessels without effects on muscular contraction is required. Furthermore, in cases of ventricular tachycardia, there is interference with coronary perfusion due to the short diastole.

Thus, a method to treat short diastole-tachyarrhythmia, and especially ventricular tachycardia, by accelerating myocardial relaxation without effects on muscular contraction is required. If administration of antiarrhythmic drugs induces onset of torsades de pointes, the only approach is to decrease the blood concentration of the antiarrhythmic drugs; however, currently sudden death cannot be prevented during the time required for reduction in the concentration.

According to J. Biochem. 131, pp. 739-743 (2002), in a myocardial tissue, actin and myosin are contractile proteins. When protein troponin and protein tropomyosin are absent, actin and myosin as contractile proteins are always in activated states, and a muscular tissue is in a contracted state. If tropomyosin is added to the muscular tissue in this state, the contracted state of the muscular tissue is not changed. However, if troponin as a Ca receptor protein is added thereto, contractile response of the muscular tissue is regulated by Ca concentration in the muscular tissue. Protein troponin is a protein complex having three components, i.e., troponin I, troponin C and troponin T. Troponin I is a contraction inhibiting protein of the muscular tissue, troponin C is a calcium ion-binding protein, and troponin T is a protein which binds to tropomyosin. When Ca ions bind to troponin C, inhibiting activity of tropoonin I on the muscular tissue is removed, i.e., myosin and actin are released from the inhibition and consequently slide over each other to give rise to contraction of the muscular tissue. Accordingly, to accelerate relaxation of the muscular tissue, it is a point how to enhance binding ability of troponin I as a muscle contraction inhibiting protein to actin-myosin complex.

Myosin is a major structural protein of muscles, which accounts for 60% of total proteins of myofibrils in skeletal muscle, and consists of two myosin heavy chains and four myosin light chains. Functions of myosin are regulated by myosin light chains, and myosin light chains have activity to bind to actin as a muscle contractile protein to play an important role in muscle contraction. Therefore, it is a point how to change activity of myosin light chains to bind to actin.

DISCLOSURE OF INVENTION

It is an object of the present invention to provide a muscle relaxation accelerating agent capable of curing or preventing megacardia particularly severe aortic value stenosis, angina pectoris caused by impairment of diastolic coronary circulation in aortic incompetence, hypertensive heart disease, idiopathic hypertrophic cardiomyopathy, valvular disease of heart, and cardiomyopathy or ventricular tachycardia which is associated with cardiac hypertrophy in the elderly or impaired myocardial relaxation by aging to show ST segment depression on electrocardiogram, by accelerating muscular relaxation and thereby ameliorating impaired myocardial relaxation to facilitate coronary circulation. Further, it is another object of the present invention to provide a therapeutic agent or prophylactic agent for torsade de pointes which is capable of curing or preventing torsade de pointes.

The present inventor has experimentally found that a 1, 4-benzothiazepine derivative represented by the formula [I]:

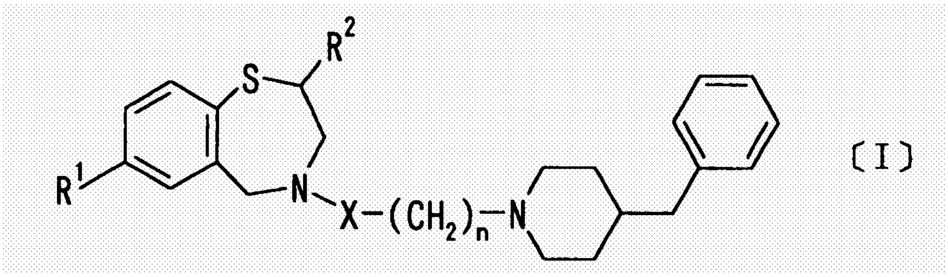

[wherein $R^1$ represents a hydrogen atom or C1-C3 lower alkoxy group, $R^2$ represents a hydrogen atom, C1-C3 lower alkoxy group or phenyl group (wherein the phenyl group may be substituted by 1 to 3 substituent groups selected from the group consisting of a hydroxyl group and a C1-C3 lower alkoxy group),

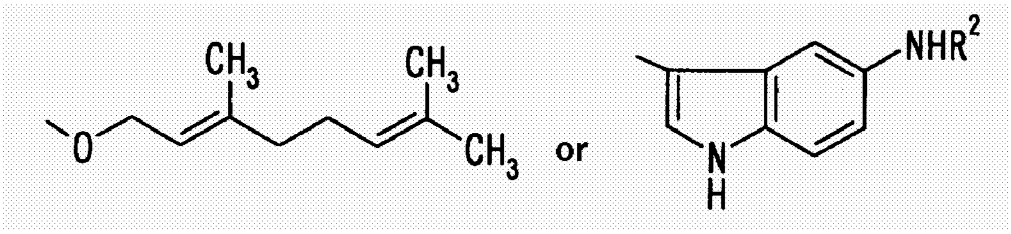

(wherein $R^3$ represents a C1-C3 acyl group), X represents -CO- or $-CH_2-$, and n represents an integer of 1 or 2], or a pharmaceutically acceptable salt thereof (hereinafter referred to as the above-mentioned 1, 4-benzothiazepine derivative or a pharmaceutically acceptable salt thereof) enhances binding function of troponin I as a muscle contraction inhibiting protein to bind to actin-tropomyosin complex in a muscle tissue, and by the enhancement of binding function of troponin I as a muscle contraction inhibiting protein to actin-tropomyosin protein complex, enhances muscle contraction inhibiting function of troponin I to thereby accelerate muscle relaxation. Further, the present inventor has found that the above-mentioned 1, 4-benzothiazepine derivative or a pharmaceutically acceptable salt thereof promotes coprecipitation of myosin light chain with actin-tropomyosin complex.

The present invention has been made based on the finding that the above-mentioned 1, 4-benzothiazepine derivative or a pharmaceutically acceptable salt thereof has function to accelerate relaxation of a muscle, i.e., muscular tissue while having no substantial influence on muscle contraction, and provides a therapeutic agent directed to promote myocardial relaxation which is fundamentally different from conventional therapeutic agents directed to promote muscle contraction, for example, conventional therapeutic agents for heart failure, and which is a therapeutic agent capable of effectively accelerating muscle relaxation substantially without affecting myocardial contraction even under a condition of calcium overload. Further, the present invention provides, for example, a therapeutic agent for left ventricular diastolic dysfunction which is capable of relieving left ventricular diastolic dysfunction in a short period of time to cure left ventricular diastolic dysfunction without affecting influence on myocardial contraction substantially, and in particular, which is effective even under a condition of calcium overload. Still further, based on the finding that the above-mentioned 1, 4-benzothiazepine derivative or a pharmaceutically acceptable salt thereof accelerates myocardial relaxation substantially without affecting myocardial contraction to improve blood flow in a microcirculatory system, the present inventor has contrived a drug for improving blood flow in a microcirculatory system. Moreover, the present inventor has found that a drug

containing the above-mentioned 1, 4-benzothiazepine derivative or a pharmaceutically acceptable salt thereof as an active ingredient accelerates myocardial relaxation substantially without affecting myocardial contraction to relieve impaired myocardial relaxation, and based thereon, has contrived a therapeutic agent for angina pectoris, in particular, a therapeutic agent for intramyocardial small vascular angina pectoris. Furthermore, based on the finding that the above-mentioned 1, 4-benzothiazepine derivative or a pharmaceutically acceptable salt thereof accelerates myocardial relaxation without affecting myocardial contraction substantially to relieve cardiomyopathy substantially, the present inventor has contrived therapeutic agents for diseases such as heart failure, hypertensive heart disease, valvular heart disease and hypertrophic cardiomyopathy which are attributable to impaired myocardial relaxation.

In addition, based on the finding that the above-mentioned 1, 4-benzothiazepine derivative or a pharmaceutically acceptable salt thereof accelerates myocardial relaxation without affecting myocardial contraction substantially to be able to rapidly relieve impaired myocardial relaxation, the present inventor has contrived a therapeutic agent for acute heart failure. Further, based on the finding that the above-mentioned 1, 4-benzothiazepine derivative or a pharmaceutically acceptable salt thereof accelerates muscle relaxation to facilitate peripheral vascular relaxation, the present inventor has contrived a therapeutic agent for hypertension. Further, the present inventor has found that the above-mentioned 1, 4-benzothiazepine derivative or a pharmaceutically acceptable salt thereof has activity capable of preventing or curing torsade de pointes although it has activity to prolong a distance from Q wave to T wave.

It is an object of the present invention to provide therapeutic agents or prophylactic agent for diseases attributable to impaired relaxation of a muscular tissue such as cardiac muscle, skeletal muscle and smooth muscle, and also provide a therapeutic agent or prophylactic agent for torsade de pointes as ventricular arrhythmia, which agents accelerate muscle relaxation substantially without affecting muscle contraction to relieve impaired myocardial relaxation in a short period of time or in a desired period of time.

In other words, the present invention resides in an accelerating agent for a muscle relaxation comprising, as an active ingredient, the above-mentioned 1, 4-benzothiazepine derivative or a pharmaceutically acceptable salt thereof, i.e., a 1, 4-benzothiazepine derivative represented by the formula [I]:

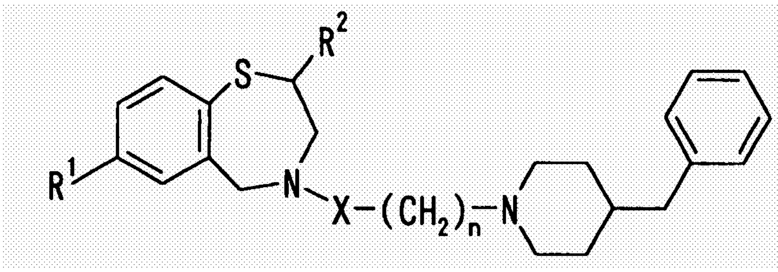

[I]

[wherein $R^1$ represents a hydrogen atom or C1-C3 lower alkoxy group, $R^2$ represents a hydrogen atom, C1-C3 lower alkoxy group or phenyl group (wherein the phenyl group may be substituted by 1 to 3 substituent groups selected from the group consisting of a hydroxyl group and a C1-C3 lower alkoxy group),

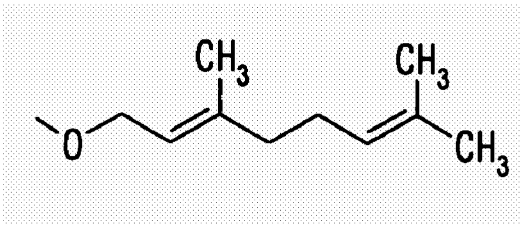

or

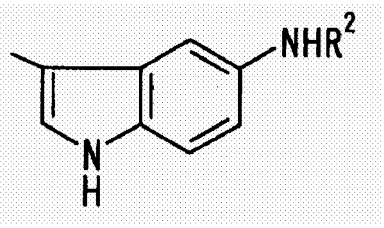

(wherein $R^3$ represents a C1-C3 acyl group), X represents -CO (carbonyl group)- or $-CH_2-$(methylene group), and n represents an integer of 1 or 2], or a pharmaceutically acceptable salt thereof. The present invention also resides in an accelerating agent for a muscle relaxation comprising the above-mentioned 1, 4-benzothiazepine derivative or a pharmaceutically acceptable salt thereof as an active ingredient, and having activity to enhance binding ability of troponin I as a muscle contraction inhibiting protein present in muscles to actin-tropomyosin complex. Further, the present invention resides in a therapeutic agent for left ventricular diastolic dysfunction comprising the above-mentioned 1, 4-benzothiazepine derivative or a pharmaceutically acceptable salt thereof as an active ingredient. Still further, the present invention resides in a therapeutic agent for left ventricular diastolic dysfunction comprising the above-mentioned 1, 4-benzothiazepine derivative or a pharmaceutically acceptable salt thereof as an active ingredient, and having activity to enhance binding ability of troponin I as a muscle contraction inhibiting protein present in muscles to actin-tropomyosin

complex. Moreover, the present invention resides in a therapeutic agent for heart failure comprising the above-mentioned 1, 4-benzothiazepine derivative or a pharmaceutically acceptable salt thereof as an active ingredient. Furthermore, the present invention resides in a therapeutic agent for heart failure comprising the above-mentioned 1, 4-benzothiazepine derivative or a pharmaceutically acceptable salt thereof as an active ingredient, and having activity to enhance binding ability of troponin I as a muscle contraction inhibiting protein present in muscles to actin-tropomyosin complex. Further, the present invention resides in a therapeutic agent for acute pulmonary edema comprising the above-mentioned 1, 4-benzothiazepine derivative or a pharmaceutically acceptable salt thereof as an active ingredient. Still further, the present invention resides in a therapeutic agent for acute pulmonary edema comprising the above-mentioned 1, 4-benzothiazepine derivative or a pharmaceutically acceptable salt thereof as an active ingredient, and having activity to enhance binding ability of troponin I as a muscle contraction inhibiting protein present in muscles to actin-tropomyosin complex. Besides, the present invention resides in a drug for improving blood flow in a microcirculation system, the drug comprising the above-mentioned 1, 4-benzothiazepine derivative or a pharmaceutically acceptable salt thereof as an active ingredient. Besides, the present invention also resides in a drug for improving blood flow in a microcirculatory system, the drug comprising the above-mentioned 1, 4-benzothiazepine derivative or a pharmaceutically acceptable salt thereof as an active ingredient, and having activity to enhance binding ability of troponin I as a muscle contraction inhibiting protein present in muscles to actin-tropomyosin complex. In addition thereto, the present invention resides in a therapeutic agent for angina pectoris comprising the above-mentioned 1, 4-benzothiazepine derivative or a pharmaceutically acceptable salt thereof as an active ingredient. In further addition thereto, the present invention resides in a therapeutic agent for angina pectoris comprising the above-mentioned 1, 4-benzothiazepine derivative or a pharmaceutically acceptable salt thereof as an active ingredient, and having activity to enhance binding ability of troponin I as a muscle contraction inhibiting protein present in muscles to actin-tropomyosin complex. Besides, the present invention resides in a drug for ameliorating cardiomyopathy comprising the above-mentioned 1, 4-benzothiazepine derivative or a pharmaceutically acceptable salt thereof as an active ingredient. Besides, the present invention also resides in a drug for ameliorating cardiomyopathy comprising the above-mentioned 1, 4-benzothiazepine derivative or a pharmaceutically acceptable salt thereof as an active ingredient, and having activity to enhance binding ability of troponin I as a muscle contraction inhibiting protein present in muscles to actin-tropomyosin complex. In addition thereto, the present invention resides in a therapeutic agent for hypertension comprising the above-mentioned 1, 4-benzothiazepine derivative or a pharmaceutically acceptable salt thereof as an active ingredient. In further addition thereto, the present invention resides in a therapeutic agent for hypertension comprising the above-mentioned 1, 4-benzothiazepine derivative or a pharmaceutically acceptable salt thereof as an active ingredient, and having activity to enhance binding ability of troponin I as a muscle contraction inhibiting protein present in muscles to actin-tropomyosin complex. Further, the present invention resides in a therapeutic agent for ventricular tachycardia comprising the above-mentioned 1, 4-benzothiazepine derivative or a pharmaceutically acceptable salt thereof as an active ingredient. Still further, the present invention resides in a therapeutic agent for ventricular tachycardia comprising the above-mentioned 1, 4-benzothiazepine derivative or a pharmaceutically acceptable salt thereof as an active ingredient, and having activity to enhance binding ability of troponin I as a muscle contraction inhibiting protein present in muscles to actin-tropomyosin complex. In further addition, the present invention resides in a therapeutic agent or prophylactic agent for torsade de pointes comprising the above-mentioned 1, 4-benzothiazepine derivative or a pharmaceutically acceptable salt thereof as an active ingredient.

In this invention, the 1,4-benzothiazepine derivative or pharmaceutically acceptable salt thereof can be 4-[3-(4-benzylpiperidin-1-yl)propionyl]-7-methoxy-2,3,4,5-tetrahydro-1,4-be nzothiazepine or a pharmaceutically acceptable salt thereof. In addition, in this invention, the muscles to be treated or prevented by using muscle relaxants are the cardiac muscle, especially the left ventricular cardiac muscle, skeletal muscles, or smooth muscles.

This invention provides the drug containing the above-mentioned 1,4-benzothiazepine derivative or pharmaceutically acceptable salt thereof, i.e., the 1,4-benzothiazepine derivative or pharmaceutically acceptable salt thereof in the general formula [1], as an active ingredient.

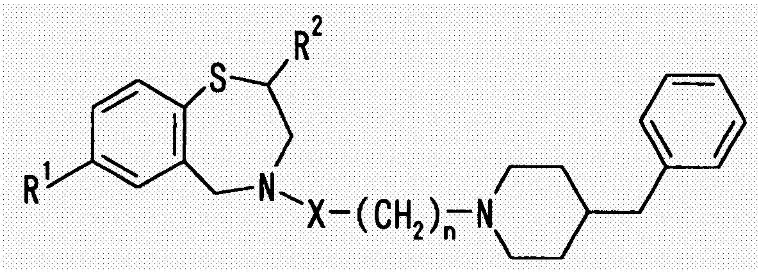

[I]

[wherein, $R^1$ represents hydrogen or an alkoxy group with 1 to 3 carbon atoms, $R^2$ represents hydrogen or an alkoxy

group with 1 to 3 carbon atoms, phenyl (wherein, phenyl can be substituted at the 1 or 3 positions with hydroxyl groups or alkoxy groups with 1 to 3 carbon atoms),

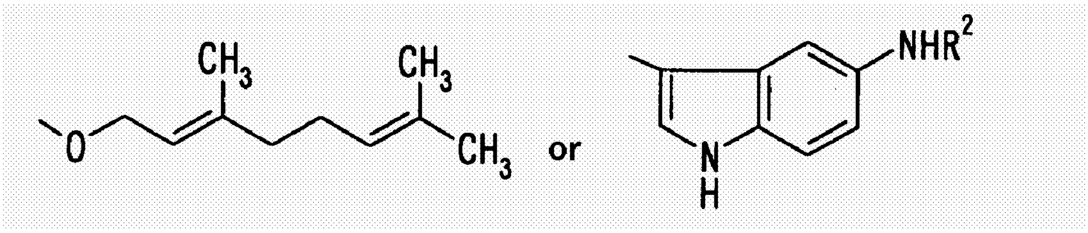

(wherein $R^3$ represents an acyl group with 1 to 3 carbon atoms), X represents -CO- (carbonyl) or $-CH_2-$ (methylene), and n = 1 or 2.]

The drug has activity to accelerate relaxation of muscles such as the cardiac muscles, skeletal muscles, and smooth muscles without affecting muscle contraction substantially. Accordingly, the drug allow the cardiac muscle to relax without affecting myocardial contraction substantially, in a short time or at a desirable time after administration; for example, the drug can dilate the left ventricle easily, and can improve coronary circulation in the cardiac muscles, and especially can improve blood flow in the microcirculatory system in the cardiac muscle. Accordingly, this invention is able to provide a therapeutic agent for dysfunction of left ventricle dilation, such as left ventricular diastolic failure, and a drug for ameliorating coronary circulation in the cardiac muscle, especially, a drug to improve microcirculation in the cardiac muscle. Furthermore, coronary perfusion is mainly performed at auxocardia of heart, and therefore facilitation of myocardial relaxation results in improvement of blood flow in the coronary circulation without affecting myocardial contraction, so drugs containing the 1,4-benzothiazepine derivative or pharmaceutically acceptable salt thereof as an active ingredient can be a therapeutic agent and a prophylactic agent for angina pectoris.

In addition, in the present invention, the drug containing the 1,4-benzothiazepine derivative or pharmaceutically acceptable salt thereof as an active ingredient has activity to accelerate relaxation of muscles such as the cardiac muscle, skeletal muscles, and smooth muscles, without affecting muscular contraction substantially, and therefore, in a short time or at a desirable time after administration, the drug relaxes muscle such as the cardiac muscles, skeletal muscles and smooth muscles to improve blood flow in small blood vessel in cardiac muscle, for example, which is associated with cardiomegaly caused by hypertension, without affecting myocardial contraction substantially, and to improve blood flow in micro blood vessel in cardiac muscle which associated with cardiomyopathy in idiopathic hypertrophic myocardiosis and subaortic stenosis, and which associated with impaired myocardial relaxation in the elderly. Accordingly, the drug is capable of a therapeutic agent and a prophylactic agent for diseases caused by these impaired myocardial relaxation, and also a therapeutic agent and a prophylactic agent for heart failure which is mainly caused by the impaired myocardial relaxation for example, heart failure caused by acute or chronic congestion of lung. Furthermore, according to the present invention, the drug containing the 1,4-benzothiazepine derivative or pharmaceutically acceptable salt thereof as an active ingredient has a function to accelerate muscles relaxation without affecting muscle contraction substantially, and can facilitate relaxation of peripheral vessel by relaxing smooth muscles rapidly without affecting muscular contraction substantially, in a short time or within a desirable time after administration, and is able to provide a therapeutic agent for hypertension. Furthermore, in this invention, the drug containing the 1,4-benzothiazepine derivative or pharmaceutically acceptable salt thereof as an active ingredient has function to accelerate relaxation of muscles without affecting muscular contraction substantially, and can easily enlarge cardiac ventricles, for example, by accelerating myocardial relaxation without affecting muscular contraction substantially in a short time or within a desirable time after administration, and is capable of a therapeutic agent for frequent arrhythmia occurring during short diastole, and especially for ventricular tachycardia. Furthermore, the drug containing the 1,4-benzothiazepine derivative or pharmaceutically acceptable salt thereof as an active ingredient has a function to accelerate muscular relaxation without affecting muscular contraction substantially, therefore, for example, the drug can treat or prevent, in a short time or within a desirable time after administration, arrhythmia developed by calcium overload in cardiac myocytes at the time of myocardial ischemia, and can serve as a therapeutic agent or a prophylactic agent for catecholamine-induced arrhythmia at the time of calcium overload in cardiac myocytes. Furthermore, the drug containing the 1,4-benzothiazepine derivative or pharmaceutically acceptable salt thereof as an active ingredient suppresses torsades de pointes induced during treatment of diseases such as arrhythmia, and enables prevention and treatment of drug-induced torsades de pointes, which is usually difficult to treat.

As stated, the drug containing the 1,4-benzothiazepine derivative or pharmaceutically acceptable salt thereof as an active ingredient can be useful for treatment and prevention of many diseases with impaired myocardial relaxation without affecting muscular contraction substantially, therefore, the drug is therapeutically extremely useful and will have a significant effect on society.

BRIEF DESCRIPTION OF FIGURES

Figure 1 is an electrocardiogram measured at 23 minutes after the start of continuous intravenous infusion of clofilium at a rate of 50 μg/kg/min and the compound at 0.2 mg/kg/min (continuous intravenous injection) in rabbits under methoxamine stimulation in Experiment A. The time course is shown on the horizontal axis of the figure. In the electrocardiogram in Figure 1, a configuration in which ventricular arrhythmia is not noted is indicated as Symbol 1.
Figure 2 is an electrocardiogram measured between approximately 25 minutes and 18 seconds and 25 minutes and 24 seconds after the start of continuous intravenous infusion of clofilium at a rate of 50 μg/kg/min (continuous intravenous injection) in rabbits under methoxamine stimulation in Control Experiment A. The time course is shown on the horizontal axis of the figure. In the electrocardiogram in Figure 2, the time point of 25 minutes and 19 seconds after administration of clofilium is indicated by Symbol 2 (arrow) and a configuration indicating torsades de pointes that appeared in the electrocardiogram is indicated by Symbol 3.
Figure 3 is an electrocardiogram measured between approximately 25 minutes and 41 seconds and 25 minutes and 48 seconds after the start of continuous intravenous infusion of clofilium at a rate of 50 μg/kg/min (continuous intravenous injection) in rabbits under methoxamine stimulation in Control Experiment A. The time course is shown on the horizontal axis of the figure. In the electrocardiogram in Figure 3, a configuration indicating torsades de pointes that appeared in the electrocardiogram is indicated by Symbol 3 and the time point 25 seconds after the configuration indicating torsades de pointes appeared in the electrocardiogram is indicated by Symbol 4 (arrow).
Figure 4 is an electrocardiogram measured between approximately 22 minutes and 26 seconds and 22 minutes and 33 seconds after the start of continuous intravenous infusion of clofilium at a rate of 50 μg/kg/min (continuous intravenous injection) in rabbits under methoxamine in Control Experiment A. The time course is shown on the horizontal axis of the figure. In the electrocardiogram in Figure 4, the time point 22 minutes and 30 seconds after administration of clofilium is indicated by Symbol 5 (arrow) and the configuration indicating torsades de pointes that subsequently appeared is indicated by Symbol 3.
Figure 5 is an electrocardiogram measured between approximately 23 minutes and 16 seconds and 23 minutes and 23 seconds after the start of continuous intravenous infusion of clofilium at a rate of 50 μg/kg/min (continuous intravenous injection) to rabbits under methoxamine stimulation in Control Experiment A. The time course is shown on the horizontal axis of the figure. In the electrocardiogram in Figure 5, the time point 49 seconds after the appearance of torsades de pointes (configuration 3) is indicated by Symbol 6 (arrow).

THE BEST MODE FOR CARRING OUT THE INVENTION

In the present invention, said 1,4-benzothiazepine derivatives or pharmaceutically acceptable salts thereof are described in Japanese Patent publication No.2,703,408 about properties and methods of manufacture of the compound which is well known as material. The Japanese Patent publication No. 2,703,408 describes the following compounds as said 1,4-benzothiazepine derivatives or pharmaceutically acceptable salts thereof;

(1) 4-[3-(4-benzylpiperidine-1-yl)propionyl]-7-methoxy-2,3,4,5-tetrahydro -1,4-benzothiazepine[namely 4-[3-[1(4-benzyl) piperidinyl] propionyl]-7-methoxy -2,3,4,5-tetrahydro-1,4-benzothiazepine],
(2) 4-[3-[1-(4-benzyl)piperidinyl]propionyl]-2-(4-methoxyphenyle)-7-methoxy -2,3,4,5-tetrahydro-1,4-benzothiazepine,
(3) 4-[1-(4-benzyl) piperidinyl] acetyl-7-methoxy-2- (4-methoxyphenyle) -2,3,4,5- tetrahydro-1,4-benzothiazepine,
(4) 4-[3-[1(4-benzyl)piperidinyl]propyl]-7-methoxy-2-3,4,5- tetrahydro -1,4-benzothiazepine,
(5) 4-[3-[1(4-benzyl) piperidinyl]propyl]-2-(4-methoxyphenyle) -7-methoxy-2-3,4,5-tetrahydro-1,4- benzothiazepine, and
(6) 4-[3-[1 (4-benzyl) piperidinyl]propionyl] -2-methoxy-2,3,4,5- tetrahydro-1,4- benzothiazepine.

The above compounds have at least a muscle relaxant effect. In this specification the following compound 4-[3-(4-benzylpiperidine-1-yl)propionyl] -7-methoxy-2,3,4,5-tetrahydro-1,4-benzothiazepine (hereinafter referred to as the present compound) among the compounds is explained.
In this invention, the 1,4-benzothiazepine derivative or pharmaceutically acceptable salt thereof contains any of 1,4-benzothiazepine derivatives or a pharmaceutically acceptable salts thereof, and has muscle relaxation acceleration activity with little effect on muscle contraction. In this invention, the drug containing any of 1, 4-benzothiazepine derivatives or pharmaceutically acceptable salts thereof is a muscle relaxation accelerating agent which facilitates muscle relaxation of the myocardium, skeletal muscles and smooth muscles in a short time or at a desirable time after administration, and without affecting muscle contraction substantially. Furthermore, the drug containing the 1,4-benzothiazepine derivative

or a pharmaceutically acceptable salt thereof is a therapeutic agent and/or a prophylactic agent for the left ventricle diastolic dysfunction, heart failure, acute pulmonary edema, angina pectoris or hypertension, and accelerates muscle relaxation in a short time or at a desirable time after administration without affecting muscle contraction substantially, and is also a improving agent of a blood flow in the microcirculation system and/or a improving agent of myocardial disorder. Furthermore, the drug containing the 1,4-benzothiazepine derivative or a pharmaceutically acceptable salt thereof is a therapeutic agent for arrhythmia caused by calcium overload in myocardial cells during myocardial ischemia, and accelerates myocardial relaxation in a short time or at a desirable time after administration without affecting myocardial contraction substantially, and is a therapeutic agent for arrhythmia induced by catecholamines such as epinephrine under conditions of calcium overload in myocardial cells during myocardial ischemia.

Both myosin and actin in muscle tissues are muscle contractile proteins, and muscle tissues are in the contracted state because myosin and actin are ordinarily activated in the absence of troponin and tropomyosin. In this condition, no changes occur in the contracted state of muscle tissues if tropomyosin is added to the muscle tissues in the contracted state, but the contraction of muscle tissues is regulated by the $Ca^{2+}$ concentration within the muscle tissues, if troponin, which is a Ca-receptor protein is added to the muscle tissues in the contracted state. Troponin is a complex of three subunits, that is, troponin I, troponin C, and troponin T. In the subunit, troponin I is a muscle contraction regulatory component of the muscle tissues, troponin C is the $Ca^{++}$ ion -binding component, and troponin T is a component which binds to tropomyosin of a muscle contraction regulatory component of the muscle tissues, and the troponin T links the troponin complex to actin and tropomyosin. Upon troponin C binding to $Ca^{++}$ ion, the muscle contraction regulatory activity of troponin I is removed and muscle tissue contraction may be caused by actin and myosin.

The actin-tropomyosin complex and the troponin C-I complex are usually detected by analyzing the precipitate obtained by ultracentrifugation of a mixture containing the actin-tropomyosin complex and the troponin C-I complex for 120 minutes at 100,000 $\times$ g at 25°C.The binding of the troponin C-I complex to the actin-tropomyosin complex in muscle tissues can be confirmed by the detected actin-tropomyosin complex and the detected troponin C-I complex. For example, when a mixture containing the actin-tropomyosin complex and the troponin C-I complex is ultracentrifuged in the presence of EGTA as a calcium chelating agent (in other words, in the absence of calcium ions), the actin-tropomyosin complex and the troponin C-I complex combine each other to precipitate together. Detection of troponin I in the precipitate using SDS-gel electrophoresis allows confirmation that the precipitate contains a complex formed by the actin-tropomyosin complex and the troponin C-I complex. Thus, if the actin-tropomyosin complex and the troponin C-I complex are precipitated, this indicates that troponin C-I is bound to the actin-tropomyosin complex and that the muscle inhibitory activity of troponin I is acting on the actin-tropomyosin complex.

The co-precipitation method using ultracentrifugation can also be used in the absence of EGTA as a calcium chelating agent (in other words, in the presence of calcium ions); however, under these conditions, troponin I or the troponin C-I complex neither bind to the actin-tropomyosin complex nor precipitate. Thus, if troponin I or the troponin C-I complex does not bind to the actin-tropomyosin complex and does not precipitate, this indicates that troponin I or the troponin C-I is not present in the actin-tropomyosin complex and that the muscle inhibitory activity of troponin I has no influence on the actin-tropomyosin complex.

The inventor has found that the precipitate is obtained by binding of troponin I to the actin-tropomyosin complex depended on the added concentration of the 1,4-benzothiazepine derivative or a pharmaceutically acceptable salt thereof when a mixture containing the actin-tropomyosin complex, troponin and the 1,4-benzothiazepine derivative or pharmaceutically acceptable salt thereof was ultracentrifuged at 100,000 $\times$ g for 120 minutes at 25°C. Thus, it was found that the 1,4-benzothiazepine derivative or pharmaceutically acceptable salt thereof enhances the bonding strength of troponin I to the actin-tropomyosin complex, resulting in the precipitate formed by binding of troponin I to the actin-tropomyosin complex. The results indicate that the 1,4-benzothiazepine derivative or a pharmaceutically acceptable salt thereof acts on troponin I, which is an inhibitor of muscle contraction, and accelerates relaxation of muscle tissues by enhancement of the action of troponin I.

A precipitate due to binding of the actin-tropomyosin complex and troponin I is usually not formed when a mixture containing the actin-tropomyosin complex, troponin and propranolol (a β-blocker) used as a therapeutic agent for heart failure is ultracentrifuged at 100,000 $\times$ g for 120 minutes 25°C. This indicates that propranolol (a β-blocker) and the 1,4-benzothiazepine derivative or pharmaceutically acceptable salt thereof have a different effect on a mixture containing the actin-tropomyosin complex and troponin, and that propranolol (a β-blocker) does not enhance the bonding strength of troponin to the actin-tropomyosin complex, unlike the 1,4-benzothiazepine derivative or pharmaceutically acceptable salt thereof.

The above-mentioned Japan Patent No. 2703408 shows that the 1,4-benzothiazepine derivative or pharmaceutically acceptable salt thereof has a function of inhibiting kinetic cell death (KD), and is available as an anti-myocardial infarction agent, and especially as a therapeutic agent and a prophylactic agent for acute myocardial infarction, or as an inhibitor of myocardial necrosis. In addition, the Patent describes a manufacturing method and various experimental data pertaining to the 1,4-benzothiazepine derivative or pharmaceutically acceptable salt thereof. The 1,4 benzothiazepine derivative has a basic nitrogen atom, so addition of an acid forms a salt at this site. The salt formed upon acid

addition is a pharmaceutically acceptable salt, and includes, for example, hydrochloride, sulfate or other inorganic salts, and citrate, maleate, fumarate, benzoate, succinate, acetate, tartrate or other organic salts.

The dosage of the 1,4 benzothiazepine derivative or pharmaceutically acceptable salt thereof used in this invention as, for example, a myocardial relaxant varies depending on the type of compound, severity of the disease, body weight of the patient, and route of administration. The drug generally can be administered at 0.1 mg to 1000 mg/day to an adult (mean weight of 60 kg), preferably 50 to 200 mg orally or parenterally (e.g. intravenous injection) administered once to three times a day, but the dosage is not limited to the above. Dosage forms for administration include, for example, powder, granules, tablets, capsules, and injections. These dosage forms can be formed in the usual manner using carrier vehicle or diluents.

Regarding the 1,4 benzothiazepine derivative used in the invention, the properties of 4-[3-(4-benzylpiperidin-1-yl)propionyl]-7-methoxy-2,3,4,5-tetrahydro-1,4-benzothiazepine (hereinafter called the compound) will be described as an example.

An ampule (injection) containing the compound is prepared using the 1-hydrochloride of the compound as an active ingredient and an isotonicity and a pH regulator: D-sorbitol can be used as the isotonicity and citric acid and sodium hydroxide can be used as the pH regulators. The preparation method is, for example, as follows: 1000 mg of D-sorbitol, 10 mg of citric acid and 40 mg of the 1-hydrochloride of the compound are dissolved in water for injection; sodium hydroxide solution and citric acid are added to the resulting 1-hydrochloride solution of the compound to adjust the pH of the solution to 3.2 to 3.3; the remaining water for injection is added with stirring to ensure dissolution; the solution is filtrated and sealed into a 20 ml ampule, and is sterilized by autoclaving. An example formulation includes 0.2% of the compound, 5% D-sorbitol, 0.5% citric acid, and 0.5% sodium hydroxide.

Furthermore, the compound is effective as a prophylactic agent and a therapeutic agent for torsades de pointes because it can inhibit manifestation of torsades de pointes, which, for example, is induced by drugs such as antiarrhythmic agents. Administration of the compound can stop the progress of torsades de pointes due to drugs which may cause prolonged QT interval or electrolyte abnormalities. Torsades de pointes usually disappears with elimination of the cause, so it is preferable that administration of the compound is accompanied by elimination of the cause when treating torsades de pointes patients by administration of the compound. For example, when treatment of arrhythmia is performed by using antiarrhythmic agents which may cause prolonged QT interval, the treatment can be carried out without inducing torsades de pointes, if the compound is used with antiarrhythmic agents. When the compound is used in combination with antiarrhythmic agents, the compound can be administered before the antiarrhythmic agents, concomitant with the antiarrhythmic agents, or after antiarrhythmic agents. In each case, administration of the compound can be used to treat arrhythmia without inducing torsades de pointes. In circumstances where the compound is administered after antiarrhythmic agents, manifestation of torsades de pointes is inhibited by administrating the compound at a specified time after administration of the antiarrhythmic agents, or after confirmation of the manifestation of torsades de pointes. Thus, the compound has the ability to prevent or treat arrhythmia, and can be administered for treatment of arrhythmia in the following way: manifestation of torsades de pointes is inhibited by administration of the compound while progress of torsades de pointes is stopped by eliminating the cause of torsades de pointes. In this invention, the total dosage of the compound for prevention and treatment of torsades de pointes is preferably 1 to 4 mg/kg, but this can be changed according to symptom as necessary. Furthermore, the administration method includes oral administration, and intramuscular and intravenous injection, but intravenous injection is preferable because of the more rapid appearance of an effect.

EMBODIMENTS

The following experiment examples are the embodiments of the invention, but the invention is not limited to the following experiment examples or descriptions.

EXPERIMENT 1

In Experiment 1, the hydrochloride of 4-[3-(4-benzylpiperidine-1-yl) propionyl]-7-methoxy-2,3,4,5-tetrahydro-1,4-benzothiazepine, the compound in the invention (hereinafter called the compound), was used as a pharmaceutically acceptable salt of the 1,4-benzothiazepine derivative. Eight-week old male Wistar rats weighing 300-330 g were used in the study. The rats were anesthetized with 1,000 mg/kg of urethane and 80 mg/kg of $\alpha$-chloralose by subperitoneal injection, and natural respiration was maintained. In this experiment, 100 mg of the compound was dissolved in 1 ml of dimethylsulfoxide (DMSO) and the resulting DMSO solution of the compound was stored at 4°C. Norepinephrine solution was prepared by dissolving 1 mg of norepinephrine in 41 $\mu$l of distilled water, at an infusion speed of 40 $\mu$g/kg/min.

Firstly, continuous infusion catheters of calcium chloride solution or norepinephrine solution containing calcium chloride were inserted into the right external jugular veins of the rats, and microchip catheters (SPC-320, Millar) were inserted into the left ventricles via the right common arteries. In addition, test drug infusion catheters were inserted into

the right femoral veins.

A 1-lead electrocardiogram and the left ventricular pressure were recorded simultaneously on a personal computer via an A/D converter. In order to determine the left ventricular end diastolic pressure, a pressure equivalent to the R wave of the electrocardiogram was measured at 20 heart beats every minute, and the mean of the pressure so measured was determined as the left ventricular end diastolic pressure, at the time of measuring. In the preparation step, blood pressure, pulse, and electrocardiogram of the rats were monitored for 15 minutes and allowed to stabilize, and then 5% dextrose solution containing calcium chloride (at a concentration based on the weight of the rat) was infused into the right external jugular vein for 20 minutes at 16.6 μl/min (9.0 mg/kg/min of calcium chloride).

Secondly, norepinephrine solution containing calcium chloride without changes in dosage was immediately injected at a rate of 40 μg/kg/min via the right external jugular vein. After the start of the administration, calcium chloride and norepinephrine were then continuously injected in the form of a norepinephrine solution containing calcium chloride. With continuous injection of calcium chloride and norepinephrine (in the form of a norepinephrine solution containing calcium chloride) via the right external jugular vein, 0.2 ml of the test drug in physiological saline was administered over 30 seconds to the first rat (weight: 300 g) as a control via the right femoral vein, 5 minutes after commencement of injection (intravenous injection) of calcium chloride and norepinephrine (in the form of a norepinephrine solution containing calcium chloride) via the right external jugular vein; this rat was defined as Control 1. Similarly to Control 1, with continuous injection of calcium chloride and norepinephrine (in the form of a norepinephrine solution containing calcium chloride) via the right external jugular vein, 0.2 ml of 1% DMSO solution as a solvent control was administered over 30 seconds to a second rat (weight: 310 g) via the right femoral vein, 5 minutes after commencement of injection (intravenous injection) via the right external jugular vein of calcium chloride and norepinephrine (in the form of a norepinephrine solution containing calcium chloride); this rat was defined as Control 2. Similarly to Controls 1 and 2, with continuous injection of calcium chloride and norepinephrine (in the form of a norepinephrine solution containing calcium chloride) via the right external jugular vein, 0.2 ml of 1% DMSO solution containing 0.3 mg/kg of the test drug was administered over 30 seconds to a third rat (weight: 310 g; Test drug 1) via the right femoral vein, 5 minutes after commencement of injection (intravenous injection) via the right external jugular vein of calcium chloride and norepinephrine (in the form of a norepinephrine solution containing calcium chloride). Similarly to Controls 1 and 3, with continuous injection of calcium chloride and norepinephrine (in the form of norepinephrine solution containing calcium chloride) via the right external jugular vein, 0.2 ml of 1% DMSO solution containing 0.3 mg/kg of the test drug was administered over 30 seconds to a fourth rat (weight: 330 g; Test drug 2) via the right femoral vein 5 minutes after commencement of injection (intravenous injection) via the right external jugular vein of calcium chloride and norepinephrine (in the form of a norepinephrine solution containing calcium chloride). In this experiment, continuous injection of calcium chloride and norepinephrine (in the form of a norepinephrine solution containing calcium chloride) via the right external jugular vein was performed even after the test drug had been administered over 30 seconds. Furthermore, in this experiment, after the test drug was injected over 30 seconds, a 1:10 dilution of the injected test drug was additionally administered to Controls 1 and 2 at 10 μl/min from commencement of test drug injection to 15 minutes after the start of administration. In Test drug 1, after 0.2 ml of 1% DMSO solution of the compound containing 0.3 mg/kg of the test drug was injected over 30 seconds, a 1% DMSO solution of the compound was additionally injected at 0.02 mg/kg/min from the commencement of test drug injection to 15 minutes after the start of administration. In Test drug 2, however, the test drug alone was injected over 30 seconds and additional injection of the test drug was not carried out. In order to determine the left ventricular end diastolic pressure of each rat, a pressure equivalent to the R wave of the electrocardiogram was measured at 20 heart beats every minute, and the mean of the pressure so measured was determined as the left ventricular end diastolic pressure, at the time of measuring. The experiment was completed 15 minutes after commencement of the test drug injection. Experimental results obtained every 5 minutes are shown in Table 1.

Table 1

| Elapsed time | Left ventricular end diastolic pressure (mmHg) | | | |
|---|---|---|---|---|
| | Control 1 (physiological saline) | Control 2 (solvent) | Test drug 1 (the compound) | Test drug 2 (the compound) |
| 30 minutes before (5 minutes before the start of $CaCl_2$ administration) | 7.7 | 7.6 | 7.5 | 8.4 |
| 25 minutes before (just after the start of $CaCl_2$ administration) | 7.5 | 7.6 | 7.8 | 8.6 |

(continued)

| Elapsed time | Left ventricular end diastolic pressure (mmHg) | | | |
|---|---|---|---|---|
| | Control 1 (physiological saline) | Control 2 (solvent) | Test drug 1 (the compound) | Test drug 2 (the compound) |
| 20 minutes before (5 minutes after the start of $CaCl_2$ administration)v | 8.4 | 8.2 | 7.9 | 8.8 |
| 15 minutes before (10 minutes after the start of $CaCl_2$ administration) | 7.5 | 7.8 | 8.6 | 9.2 |
| 10 minutes before (15 minutes after the start of $CaCl_2$ administration) | 8.5 | 8.4 | 8.5 | 9.0 |
| 5 minutes before (just before the start of intravenous injection of norepinephrine) | 8.6 | 8.6 | 8.6 | 9.3 |
| 0 minutes (just before the start of test drug administration) | 7.8 | 8.8 | 8.9 | 9.6 |
| 5 minutes after (5 minutes after the start of test drug administration) | 11.9 | 10.1 | 10.6 | 13.8 |
| 10 minutes after (10 minutes after the start of test drug administration) | 30.4 | 37.5 | 12.5 | 16.4 |
| 15 minutes after (15 minutes after the start of test drug administration) | 47.3 | 49.4 | 11.8 | 15.3 |
| (Note 1) "before" in "30 minutes before" - "5 minutes before" means before the start of test drug administration (injection).<br>(Note 2) "0 minutes" - "15 minutes" mean an elapsed time of 0 minutes to 15 minutes after the start of intravenous injection of the test drug.<br>(Note 3) the name of the test drug is given in parentheses below Control 1, Control 2, Test drug 1 and Test drug 2. The test drug used in the invention is described as "the compound" in parentheses. | | | | |

This experiment was performed at 20 to 25°C. In this experiment, the left ventricular diastolic pressures in Controls 1 and 2 were 7.7 to 8.6 mmHg and 7.6 to 8.6 mmHg, respectively, and these pressures in Test drugs 1 and 2 were 7.5 to 8.6 mmHg and 8.4 to 9.3 mmHg, respectively, from after the calcium chloride injection to just before intravenous injection of norepinephrine. The left ventricular diastolic pressure from after the calcium chloride injection to just before the intravenous injection of norepinephrine was almost the same in Test drugs 1 and 2 and Controls 1 and 2, respectively. However, the left ventricular diastolic pressure from 15 minutes (10 minutes after commencement of intravenous injection of the test drug) to 20 minutes after commencement of intravenous injection of norepinephrine (15 minutes after commencement of intravenous injection of the test drug) increased from 30.4 to 47.3 mmHg in Control 1 and 37.5 to 49.4

mmHg in Control 2, and diastolic failure of the left ventricle developed in both Controls. In contrast, in Test drug 1 the left ventricular diastolic pressure elevated to 12.5 mmHg 15 minutes after commencement of intravenous injection of norepinephrine (10 minutes after commencement of intravenous injection of the test drug), but the left ventricular diastolic pressure decreased to 11.8 mmHg 15 minutes after commencement of intravenous injection of the test drug compound (20 minutes after commencement of intravenous injection of norepinephrine). The left ventricular diastolic pressure in Test drug 1 was less than half those in Controls 1 and 2 at the same time point, which indicates that intravenous injection of the test drug does not cause diastolic failure of the left ventricle. Similarly, in Test drug 2, the left ventricular diastolic pressure elevated to 16.4 mmHg 15 minutes after commencement of intravenous injection of norepinephrine (10 minutes after commencement of intravenous injection of the test drug), but the left ventricular diastolic pressure decreased to 15.3 mmHg 15 minutes after commencement of intravenous injection of the test drug compound (20 minutes after commencement of intravenous injection of norepinephrine). The left ventricular diastolic pressure in Test drug 2 was less than half those in Controls 1 and 2 at the same time point, which indicates that intravenous injection of the compound of the test drug does not cause diastolic failure of the left ventricle.

Based on the above results for Test drugs 1 and 2, additional injection of the test drug makes it easier to examine the effect of the test drug in decreasing the left ventricular diastolic pressure. Furthermore, the results prove that the test drug is effective as a therapeutic agent and a prophylactic agent for diastolic failure of the left ventricle.

EXPERIMENT 2

Effect of the compound on blood pressure

Eight-week old male Wistar rats weighing 310-330 g were used in this experiment. The rats were anesthetized with 1,000 mg/kg of urethane and 80 mg/kg of a-chloralose by subperitoneal injection, and natural respiration was maintained. In this experiment, 100 mg of the compound was dissolved in 1 ml of dimethylsulfoxide (DMSO) and the resulting DMSO solution of the compound was stored at 4°C. Norepinephrine solution was prepared by dissolving 1 mg of norepinephrine in 41 μl of distilled water.

Similarly to Experiment 1, this experiment was performed at 20 to 25°C. Furthermore, similarly to Experiment 1, continuous infusion catheters of calcium chloride solution or norepinephrine solution containing calcium chloride were inserted into the right external jugular veins of the rats, and microchip catheters (SPC-320, Millar) were inserted into the aorta via the right common arteries.

A 1-lead electrocardiogram and the systolic pressure and diastolic pressure were recorded on a personal computer via an A/D converter. In the preparation step, blood pressure, pulse, and electrocardiogram of the rats were monitored for 15 minutes and allowed to stabilize, and then 5% dextrose solution containing calcium chloride (at a concentration based on the weight of the rat) was infused into the right external jugular vein for 25 minutes at 16.6 μl/min (9.0 mg/kg/min of calcium chloride).

Secondly, norepinephrine solution containing calcium chloride without changes in the dosage regimen was immediately injected at a rate of 40 μg/kg/min (in the form of a norepinephrine solution containing calcium chloride). Five minutes after commencement of injection (intravenous injection) of norepinephrine, continuous injection of calcium chloride and norepinephrine in the form of a norepinephrine solution containing calcium chloride was started, and 0.2 ml of 1% DMSO solution as a solvent control was administered over 30 seconds to the first rat (weight: 310 g) via the right femoral vein; this rat was defined as Control 3. After the solvent control was injected, 1% DMSO solution as a solvent control was additionally administered to Control 3 at 10 μl/min from commencement of solvent control injection to 15 minutes after the start of administration. Similarly to Control 3, 5 minutes after commencement of injection (intravenous injection) of norepinephrine in the form of a norepinephrine solution containing the calcium chloride, continuous injection of the calcium chloride and norepinephrine in the form of a norepinephrine solution containing calcium chloride was started, and 0.2 ml of 1% DMSO solution containing 0.3 mg/kg of the compound was administered over 30 seconds to a second rat (weight: 330 g; referred to as Test drug 3), and 1% DMSO solution containing 0.02 mg/kg of the test drug was then administered for 14 minutes at a rate of 0.02 mg/kg/min. Systolic pressure (mmHg) and diastolic pressure (mmHg) of each rat were measured at 20 heart beats every 2 minutes for 14 minutes after commencement of intravenous injection of the test drug. Mean blood pressure was calculated from the measured systolic pressure (mmHg) and diastolic pressure (mmHg) according to the following formula: mean blood pressure = [(systolic pressure + diastolic pressure) × 1/2]. The results are shown in Table 2.

Table 2

| Elapsed time | Mean blood pressure (mmHg) | |
|---|---|---|
| | Control 3(solvent) | Test drug 3 (the compound) |
| 30 minutes before (5 minutes before the start of $CaCl_2$ administration) | 110 | 122 |
| 5 minutes before (just before the start of intravenous injection of norepinephrine) | 121 | 125 |
| 0 minutes (just before the start of test drug administration) | 135 | 136 |
| 2 minutes after (2 minutes after the start of test drug administration) | 147 | 149 |
| 4 minutes after (4 minutes after the start of test drug administration) | 160 | 162 |
| 6 minutes after (6 minutes after the start of test drug administration) | 160 | 141 |
| 8 minutes after (8 minutes after the start of test drug administration) | 160 | 139 |
| 10 minutes after (10 minutes after the start of test drug administration) | 161 | 135 |
| 12 minutes after (12 minutes after the start of test drug administration) | 158 | 136 |
| 14 minutes after (14 minutes after the start of test drug administration) | 160 | 134 |
| (Note 1) "before" in "30 minutes before" - "5 minutes before" means "before the start of test drug administration (injection)".<br>(Note 2) "0 minutes" - "14 minutes" mean an elapsed time of 0 minutes to 14 minutes after the start of intravenous injection of the test drug.<br>(Note 3) the name of the test drug is given in parentheses below Control 3 and Test drug 3. The test drug used in the invention is described as "the compound" in parentheses. | | |

In this Experiment, the mean blood pressure in Control 3 increased to approximately 160 mmHg and varied from 4 minutes after commencement of intravenous injection (infusion) of norepinephrine. On the contrary, the mean blood pressure of Test drug 3 which was administered the compound reached to 162 mmHg. However, thereafter it decreased and remained between 136 and 134 mmHg for 10 to 14 minutes after commencement of intravenous injection (infusion) of norepinephrine and a decrease in mean blood pressure was evident. This result indicates that the compound is effective as a therapeutic agent for hypertension.

EXPERIMENT 3

An investigation of the effects of the test drug in the invention on left ventricular diastolic function using tissue Doppler imaging (the Doppler method)

In this experiment on the invention, two 9-week old male Wistar rats weighing 310 and 320 g were used. The rats were anesthetized with 1,000 mg/kg of urethane and 80 mg/kg of α-chloralose by subperitoneal injection, and natural respiration was maintained. In this experiment, 100 mg of the compound was dissolved in 1 ml of dimethylsulfoxide (DMSO) and the resulting DMSO solution was stored at 4°C. Norepinephrine solution was prepared by dissolving 1 mg of norepinephrine in 41 μl of distilled water.

Similarly to Test drug 1, this study was performed at room temperature of 20 to 25°C, and also similarly to Test drug 1, continuous infusion catheters of calcium chloride solution or norepinephrine solution containing calcium chloride were inserted into the right external jugular vein and microchip catheters (SPC-320, Millar) were inserted into the aorta via the right common arteries.

A 1-lead electrocardiogram was recorded, and blood pressure, pulse, and electrocardiogram of the rats were monitored for 15 minutes and allowed to stabilize. In the preparation step, calcium chloride dissolved in 5% dextrose

solution was infused into the right external jugular vein for 25 minutes at 16.6 μl/min (9.0 mg/kg/min of calcium chloride).

Secondly, immediate injection (intravenous injection) of a solution containing calcium chloride and norepinephrine without changes in dosage was started via the right external jugular vein at a rate of 40 μg/kg/min. After the start of administration, calcium chloride and norepinephrine were continuously injected in the form of a norepinephrine solution containing calcium chloride. Furthermore, with continuous injection of calcium chloride and norepinephrine (in the form of a norepinephrine solution containing calcium chloride), 0.2 ml of 1% DSMO was administered alone over 30 seconds to the first rat (weight: 310 g) as a control via the right femoral vein, 5 minutes after commencement of intravenous injection of calcium chloride and norepinephrine (in the form of a norepinephrine solution containing calcium chloride); this rat was defined as Control 4. Similarly to Control 4, with continuous injection of calcium chloride and norepinephrine (in the form of a norepinephrine solution containing calcium chloride) 0.2 ml of 1% DSMO solution containing 0.3 mg/kg of the test drug in the invention was administered over 30 seconds to a second rat (weight: 320 g; Test drug 3) via its right femoral vein, followed by administration for 20 minutes at 0.02 mg/kg/min, 5 minutes after intravenous injection of calcium chloride and norepinephrine (in the form of a norepinephrine solution containing calcium chloride). The left ventricular diastolic function of each rat was examined using tissue Doppler ultrasonography. Ultrasonic diagnostic equipment (Toshiba Powervision SSA-380APSK-70LT, Toshiba Corporation) was used and the rats were examined using ultrasound at 10 MHz. Firstly, the precordial region of each rat was shaved, and an ECHO probe was placed on the cardiac apex. The long axis of the left ventricle of the cardiac apex was imaged, a sample volume for pulsed-wave Doppler was established at the base of the posterior mitral leaflet, and the velocity of the left ventricular posterior wall motion [Ea wave (m/sec)] was measured at diastole as the left ventricular diastolic function. In this experiment, the left ventricular diastolic function is defined as the ratio of the velocity of the left ventricular wall motion at diastole before intravenous injection of norepinephrine (before administration; i.e. a normal left ventricular wall) determined using tissue Doppler imaging, or the ratio of the velocity of the left ventricular wall motion at diastole after intravenous injection of norepinephrine (after administration). The results are shown in Table 3.

Table 3

| Elapsed time | Ratio of the velocity of the left ventricular wall motion at diastole | |
|---|---|---|
| | Control 4 (solvent) | Test drug 4 (The compound) |
| 5 minutes before (just before the start of intravenous injection of norepinephrine) | 1.00 | 1.00 |
| 0 minutes (just before the start of intravenous injection of the test drug) | 1.00 | 1.00 |
| 5 minutes after (5 minutes after the start of intravenous injection of the test drug) | 0.85 | 1.02 |
| 10 minutes after (10 minutes after the start of intravenous injection of the test drug) | 0.75 | 1.04 |
| 15 minutes after (15 minutes after the start of intravenous injection of the test drug) | 0.70 | 1.00 |
| 20 minutes after (20 minutes after the start of intravenous injection of the test drug) | 0.60 | 1.00 |
| 25 minutes after (25 minutes after the start of intravenous injection of the test drug) | 0.55 | 0.94 |
| 30 minutes after (30 minutes after the start of intravenous injection of the test drug) | 0.51 | 0.95 |
| (Note 1) "before" in "5 minutes before" means "before the start of intravenous injection of the test drug".<br>(Note 2) "0 minutes" - "30 minutes" mean an elapsed time of 0 minutes to 30 minutes after the start of intravenous injection of the test drug.<br>(Note 3) the name of the test drug is given in parentheses below Control 4 and Test drug 4. The test drug in the invention is described as "the compound" in parentheses. | | |

In this experiment, the ratio of the velocity of the left ventricular wall motion at diastole in Control 4 to the velocity of the normal left ventricular wall motion at diastole (Ea wave) decreased to 0.85 5 minutes after the start of intravenous injection, and the ratio of the velocity of the left ventricular wall motion at diastole in Control 4 (Ea wave) to the velocity

of the normal left ventricular wall motion at diastole (Ea wave) decreased to 0.51 30 minutes after the start of intravenous injection. In contrast, in Test drug 3, the ratio of the velocity of the left ventricular wall motion at diastole (Ea wave) to the velocity of the normal left ventricular wall motion at diastole (Ea wave) remained at 0.95, showing little variability. The small change in this ratio suggests that the left ventricular wall motion is slow, since the velocity of the left ventricular wall motion indicates the speed of ventricular wall motion per unit time. The result also indicates that the compound in the invention may be effective as a therapeutic agent and a prophylactic agent for heart failure caused by left ventricular diastolic dysfunction.

EXPERIMENT 4

Measuring reagent of binding of troponin I to the actin-tropomyosin complex

Swine muscle-derived actin, chicken gizzard-derived tropomyosin and swine myocardium-derived troponin used in this experiment were purchased from Sigma-Aldrich Corporation, and other unspecified reagents used in this experiment were purchased from Wako Pure Chemical Industries Ltd.

A 500 μl sample was prepared by adding 4.2 μg of actin, 2.1 μg of tropomyosin and 14 μg of troponin to 500 μl of a reaction solution containing 60 mM KCl, 20 mM MOPS (3-morpholinopropanesulfonic acid), 2 mM of $MgCl_2$, 0.05 μg of pepstatin A, and 15 mM 2-mercaptoethanol. After centrifugation of this sample at 2,000 × g for 10 minutes at 25°C, the precipitate was removed and the resulting supernatant was distributed to separate test tubes containing 10 mM of EGTA, and each tube was determined as a test sample.

In this experiment, the test sample in the first test tube did not contain the compound, i.e. the sample was a reaction solution with a 0 mol concentration of the compound; the test sample in the second test tube was a reaction solution with a $10^{-4}$ mol concentration of the compound; the test sample in the third test tube was a reaction solution with a $10^{-5}$ mol concentration of the compound; the test sample in the forth test tube was a reaction solution with a $10^{-6}$ mol concentration of the compound; and the test sample in the fifth test tube was a reaction solution with a $10^{-7}$ mol concentration of the compound. Each test sample was reacted for 120 minutes at 25°C and centrifuged at 100,000 × g for 120 minutes at 25°C, and the supernatants were removed. After reaction solution was gently added to the removed precipitate so as not to suspend the precipitate, the precipitate was again removed by suction, washed, and determined as a test precipitate.

A sample buffer for polyacrylamide gel electrophoresis (SDS-PAGE) was added to each washed test precipitate and mixed well to suspend the test precipitate. The resulting suspension was electrophoresized on a gel (PAG MINI "No.1" 10/20) for about 1 hour at 40 mA. After electrophoresis, the suspension was silver-stained using "Daiichi" 2D-silver staining reagent (Daiichi Pure Chemicals Co., Ltd), and washed with distilled water and dried. The amount of precipitate in each silver-stained protein band obtained by gel electrophoresis was quantitated using a densitometer. The amino-acid sequence of the peptide revealed that protein in the quantitated precipitate in the protein band was troponin I. The protein band from gel electrophoresis was stained using a reagent for mass spectrometry (silver staining reagent for mass spectrometry, Wako Pure Chemical Industries Ltd.), and a peptide fragment obtained from the stained protein band was analyzed by a high performance liquid chromatography apparatus (MAGIC2002, Michrom BioResources, Inc, USA).,and the amino-acid sequence of the peptide of the protein band from gel electrophoresis was determined as result. This analysis showed that the amino-acid sequence of the peptide from the protein band was IDAAEEEKYDMEIK, and the protein was identified as troponin I.

The amounts of precipitate of troponin I in the second to fifth test precipitates were calculated as ratios of the amount of precipitate of troponin I in the first test precipitate, which was obtained without the compound (the concentration of the compound was 0 M).

COMPARISON EXAMPLE

The affinity of troponin I for the actin-tropomyosin complex was measured using the same method as in Experiment 4, but using propranolol (a β-blocker which is used as a therapeutic agent for heart failure) for comparison with the compound in the invention.

A 500 μl sample was prepared by adding 4.2 μg of actin, 2.1 μg of tropomyosin and 14 μg of troponin I to 500 μl of a reaction solution containing 60 mM KCl, 20 mM MOPS (3-morpholinopropanesulfonic acid), 2 mM $MgCl_2$, 0.05 μg of pepstatin A, and 15 mM 2-mercaptoethanol. This sample was centrifuged at 2,000 × g for 10 minutes at 25°C, precipitate was removed, and the resulting supernatant was distributed to separate test tubes containing 10 mM of EGTA, and each was determined as a test sample.

In the comparison experiment, the comparison sample in the first test tube was a reaction solution containing 0 mol concentration of propanolol; the comparison sample in the second test tube was a reaction solution containing $10^{-4}$ mol concentration of propanolol; the comparison sample in the third test tube was a reaction solution containing $10^{-5}$ mol concentration of propanolol; the comparison sample in the fourth test tube was a reaction solution containing

$10^{-6}$ mol concentration of propanolol; and the comparison sample in the fifth test tube was a reaction solution containing $10^{-7}$ mol concentration of propanolol. Each sample was reacted for 120 minutes at 25°C and then centrifuged at 100,000 × g for 120 minutes at 25°C, after which the supernatants were removed. Reaction solution was gently added so as not to suspend the precipitate, and the precipitate was again removed by suction, washed, and determined as a test precipitate.

A sample buffer for polyacrylamide gel electrophoresis (SDS-PAGE) was added to each washed comparison test precipitate and mixed well, the comparison precipitate was suspended, and the resulting suspension was electrophoresized on a gel (PAG MINI "No.1" 10/20) for about 1 hour at 40 mA. After electrophoresis, the suspension was silver-stained using "Daiichi" 2D-silver staining reagent (Daiichi Pure Chemicals Co., Ltd), and washed with distilled water and dried. The amount of precipitate in each silver-stained protein band obtained in gel electrophoresis was quantitated using a densitometer.

The amount of precipitate of troponin I in the second to fifth comparison test precipitates was calculated as a ratio of the amount of precipitate of troponin I in the comparison test precipitate from the sample that did not contain propranolol (a propranolol concentration of 0 M).

Table 4 shows a comparison of the amount of precipitate of troponin I in the test sample in Experiment 4, in which the compound in the invention was used, and the amount of precipitate of troponin I in the comparison sample, in which propranolol was used.

Table 4

| Test Drug 5 | | Comparison example | |
|---|---|---|---|
| Compound concentration (mol) | Precipitate of troponin I | Propranolol Concentration (mol) | Precipitate of troponin I |
| 0 | 1.0 | 0 | 1.0 |
| $10^{-7}$ | 1.2 | $10^{-7}$ | 0.9 |
| $10^{-6}$ | 1.8 | $10^{-6}$ | 1.1 |
| $10^{-5}$ | 2.2 | $10^{-5}$ | 0.9 |
| $10^{-4}$ | 2.8 | $10^{-4}$ | 1.1 |
| Note) In Table 4, the compound in the invention is described as "the compound". | | | |

The amount of precipitate of troponin I for each concentration of the 1-hydrochloride of the compound used in Test Drug 5 samples and the amount of precipitate of troponin I for each concentration of propranolol used in the comparison samples are shown in Table 4. As seen for the Test Drug 5 samples in Table 4, the amount of precipitate of troponin I increased from 1.2 to 2.8 when the compound concentration (mol) increased from $10^{-7}$ to $10^{-4}$ M. However, with an increase in the concentration of propranolol from $10^{-7}$ to $10^{-4}$ M the amount of precipitate of troponin I ranged between 0.9 and 1.1, and showed little change in the comparison examples, in which propranolol was used. The increase in the amount of troponin I with an increase in the compound concentration, as seen in the Test Drug 5 examples in Table 4, resulted from an increase in binding of troponin I and the actin-tropomyosin complex. This suggests that the compound relaxes muscles through troponin I by increasing the amount of troponin I bound to the actin-tropomyosin complex; hence, the results indicate that the compound facilitates muscle relaxation through troponin I.

## EXPERIMENT 5

In this experiment, the effects of the compound were studied on the amount of precipitate of myosin light chains, which is a component of the troponin test sample used in Experiment 4.

In this experiment, the sample precipitate was prepared according to the procedure used in Experiment 4, except that in this experiment: (1) the test sample did not contain calcium ions ($Ca^{2+}$) and the compound, and the test precipitate was prepared in the manner used in Experiment 4 (Test 1), (2) the concentrations (mol) of calcium ions ($Ca^{2+}$) and the compound in the test sample used in Experiment 4 were determined as $10^{-5}$ and 0, respectively, and the test precipitate was prepared in the manner used in Experiment 4 (Test 2), (3) the concentrations (mol) of calcium ions ($Ca^{2+}$) and the compound in the test sample used in Experiment 4 were determined as 0 and $10^{-3}$, respectively, and the test precipitate was prepared in the manner used in Experiment 4 (Test 3), and (4) the concentrations (mol) of calcium ions ($Ca^{2+}$) and the compound in the test sample used in Experiment 4 were determined as $10^{-5}$ and 0, respectively, and the test precipitate was prepared in the manner used in Experiment 4 (Test 4)

A sample buffer for polyacrylamide gel electrophoresis (SDS-PAGE) was added to each washed test precipitate,

which was prepared in the manner described above, and mixed well to suspend the test precipitate. The resulting suspension was electrophoresed on a gel (PAG MINI "No.1" 10/20) for about 1 hour at 40 mA. After electrophoresis, the suspension was silver-stained using "Daiichi" 2D-silver staining reagent (Daiichi Pure Chemicals Co., Ltd), and washed with distilled water and dried. The amount of precipitate in each silver-stained protein band obtained by gel electrophoresis was quantitated using a densitometer. The amino-acid sequence of the peptide revealed that the protein in the quantitated precipitate giving the protein band was myosin light chains. The protein band from gel electrophoresis was stained using a reagent for mass spectrometry (silver staining reagent for mass spectrometry, Wako Pure Chemical Industries Ltd.), and a peptide fragment obtained from the protein that gave the band in gel electrophoresis was analyzed using high performance liquid chromatography (MAGIC2002, Michrom BioResources, Inc, USA). This analysis showed that the amino-acid sequence of the peptide from the protein band was HVLATLGEK and ITLSQVGDVLR, and the protein was identified as myosin light chains.

The amounts of precipitate obtained in Experiments 1 to 4 are shown in Table 5.

Table 5

| Test Number | $Ca^{2+}$ concentration (mol) | Concentration of the compound (mol) | The amount of precipitate of myosin light chains |
|---|---|---|---|
| 1 | 0 | 0 | 1.0 |
| 2 | $10^{-5}$ | 0 | 0.6 |
| 3 | 0 | $10^{-3}$ | 1.5 |
| 4 | $10^{-5}$ | $10^{-3}$ | 1.4 |
| (Note) The amount of precipitate of myosin light chains is described as the ratio to the amount of precipitate in Test 1, which is defined as 1.0.<br>(Note) In Table 5, the compound in the invention is described as "the compound". | | | |

In this Experiment, the amount of precipitate of myosin light chains increased in the test precipitates (Tests 1 and 3) when calcium ions were absent (relaxed state), compared to the test precipitates (Tests 2 and 4) when the calcium ion ($Ca^{2+}$) concentration was $10^{-5}$ mol concentration. In addition, the amount of precipitate of myosin light chains increased in the test precipitate when the concentration of the compound was $10^{-3}$ mol concentration (Tests 3 and 4), compared to the test precipitate when the compound was absent (Tests 1 and 2). Furthermore, the amount of precipitate of myosin light chains in the test precipitate when the concentration of the compound was $10^{-3}$ mol concentration (Tests 3 & 4) was slightly larger that for the test precipitate without calcium ions than for that with calcium ions, although the amounts of these precipitates were almost equal. Thus, the results indicate that the presence of the compound increases the amount of precipitate of myosin light chains, and suggests that the effect with a 0 mol concentration of calcium ion concentration is the same as that with a $10^{-5}$ mol concentration of calcium ion concentration. In conclusion, these results indicate that the compound enhances muscular relaxation, regardless of the presence or absence of calcium ions.

EXPERIMENT 6

Measurement of the affinity of troponin I and the actin-tropomyosin complex

A solution of 0.4 μg of troponin I (Calbiochem Inc) and 0.54 μg of troponin C (Abcam PLC) was added to 23 μl of distilled water, distributed to 4 test tubes, and centrifuged at 100,000 g for 1 hour (HITACHI Himac 120FC), and 20 μl of the resulting supernatant was collected and stored at 4°C. Then, 50 μl of 600 mM KCl solution, 50 μl of 200 mM MOPS (3-morpholinopropanesulfonic acid) [3-N (morpholino) propanesulfonic acid] (DOJINDO) solution, 50 μl of 20 mM $MgCl_2$ solution, 5 μl of 10 mg/ml pepstatin A (SIGMA) solution, and 50 μl of 150 mM 2-mercaptoethanol (SIGMA) were added as a reaction solution to 99.4 μl of distilled water, and distributed to another 4 test tubes. Next, 3.2 μl of 1 mg/ml actin (SIGMA) and 2.4 μl of 1 mg/ml tropomyosin (SIGMA) were added and mixed, and the mixture was then centrifuged at 2000 g for 5 minutes at 25°C using a centrifuge (HITACHI Himac CF7D2). The resulting supernatant was put into 4 new test tubes, 50 μl of 50 mM ATP (adenosine 5'-triphosphate) solution was added, and the solution was incubated at 25°C with mixing for 30 minutes.

Next, 20 μl of the 100,000 g supernatant of troponin I and troponin C which was previously prepared was added to each test tube, and incubated at 25°C with mixing for 30 minutes. Distilled water alone or 50 μl of a test solution in which the final concentration of the compound was adjusted to $10^{-7}$, $10^{-6}$ and $10^{-5}$ M was added, and the resulting solution was incubated at 25°C with mixing for 30 minutes.

Subsequently, the final volume was adjusted to 500 μl/tube by adding 50 μl of a solution in which the final

calcium concentration was adjusted to $10^{-6}$ M by mixing $Ca^{2+}$ solution and EGTA solution. The solution was incubated at 25°C with intermittent mixing for 60 minutes, and then centrifuged at 100,000 g for 120 minutes at 25°C using a centrifuge (HITACHI Himac 120FC: brand name).

After centrifugation, the supernatant was removed, 1 ml of the reaction liquid (excluding $Ca^{2+}$ and the drug) was gently added so as not to suspend any precipitate, and the precipitate was again removed by suction; the procedure was repeated twice and the precipitate was washed 3 times in total.

The washing solution was removed completely, 20 μl of a sample buffer for polyacrylamide gel electrophoresis (SDS-PAGE) was added and mixed well, the precipitate was suspended and heated for 5 minutes at 95°C, and then poured into a PAG MINI "No.1" 10/20 gel (Daiichi Pure Chemicals Co., Ltd) and electrophoresized for about 1 hour at 40 mA. After electrophoresis, the suspension was silver-stained using "Daiichi" 2D-silver staining reagent (Daiichi Pure Chemicals Co., Ltd), and washed and dried. Finally, the suspension was scanned (EPSON ES-8500 scanner: brand name) and analyzed (Image J software: brand name). The results are shown in Table 6.

Table 6. Amount of precipitate of troponin I

| Test tube No. | Condition | Ratio |
|---|---|---|
| 1 | Ca $10^{-6}$ M the compound (-) | 1.0 |
| 2 | Ca $10^{-6}$ M the compound ($10^{-7}$ M) | 1.4 |
| 3 | Ca $10^{-6}$ M the compound ($10^{-6}$ M) | 3.4 |
| 4 | Ca $10^{-6}$ M the compound ($10^{-5}$ M) | 5.4 |

In this experiment, at a calcium concentration in the test tube of $10^{-6}$ M, the amount of troponin I precipitated in the absence of the compound was set at 1.0. Relatively, the amount of co-precipitation in the presence of $10^{-7}$, $10^{-6}$ and $10^{-5}$ M concentrations of the compound was 1.4, 3.4, and 5.4, respectively; therefore, it was confirmed that the amount of troponin I precipitate increased with an increase in the compound concentration. The actin used in this experiment was swine skeletal muscle-derived actin with a molecular weight of 43 kDa, and was purchased from SIGMA. Tropomyosin used in this experiment was gizzard-derived tropomyosin with a molecular weight of 36 kDa, and was purchased from SIGMA. Troponin I used in this experiment was human myocardium-derived troponin I with a molecular weight of 24 kDa, and was purchased from Calbiochem Inc. Troponin C used in this experiment was human myocardium recombinant-derived troponin C with a molecular weight of 18 kDa, and was purchased from Abcam PLC. These proteins were separated by SDS-polyacrylamide gel electrophoresis and each band was clearly identifiable.

In this experiment, a mixed solution containing 0.1 to 1.2 μg of troponin I and 0.2 to 1.6 μg of troponin C, which are aggregating components that can be obtained by ultracentrifugation, 3.2 μl of an actin solution with a concentration of 0.5 to 3 mg/ml, 2.4 μl of a tropomyosin solution with a concentration of 0.5 to 3 mg/ml, and a solution containing 4 to 6 mM ATP was used. The mixed solution was, for example, adjusted to 500 microliters in total before the reaction; therefore, these drugs can be used at an adjusted concentration within the concentration ranges above.

EXPERIMENT 7

Measurement of the affinity troponin I and the actin-tropomyosin complex

A solution containing 0.4 μg of troponin I (Calbiochem Inc) and 0.54 μg of troponin C (Abcam PLC) was added to 23 μl of distilled water, distributed to 4 test tubes and centrifuged at 100,000 g for 1 hour (HITACHI Himac 120FC), and 20 μl of the resulting supernatant was collected and stored at 4°C. Then, 50 μl of 600 mM KCl solution, 50 μl of 200 mM MOPS (3-morpholinopropanesulfonic acid, DOJINDO) solution, 50μl of 20 mM $MgCl_2$ solution, 5μl of 10 μg/ml pepstatin A (SIGMA) solution, and 50 μl of 150 mM 2-mercaptoethanol (SIGMA) were added as a reaction solution to 99.4 μl of distilled water, and distributed to another 4 test tubes. Next, 3.2 μl of 1 mg/ml actin (SIGMA) and 2.4 μl of 1 mg/ml tropomyosin (SIGMA) were added and mixed, and the mixture was then centrifuged at 2,000 g for 5 minutes at 25°C using a centrifuge (HITACHI Himac CF7D2). The resulting supernatant was put into new 4 test tubes, 50 μl of 50 mM ATP solution was added, and the solution was then incubated at 25°C with mixing for 30 minutes. Next, 20 μl of the 100,000 g supernatant of troponin I and troponin C which was previously prepared was added to each test tube, and incubated at 25°C with mixing for 30 minutes. Then, 50 μl of the compound at a final adjusted concentration of $10^{-5}$ M was added to each test tube as the test solution, and incubated at 25°C with mixing for 30 minutes.

Subsequently, the final amount was adjusted to 500 μl/tube by adding 50 μl of solution in which the final calcium concentration was adjusted to $10^{-8}$, $10^{-7}$, $10^{-6}$, or $10^{-5}$ M by mixing a $Ca^{2+}$ solution and an EGTA solution. The solution was incubated at 25°C with intermittent mixing for 60 minutes, and centrifuged at 100,000 g for 120 minutes at 25°C using a centrifuge (HITACHI Himac 120FC). After centrifugation, the supernatant was removed, 1 ml of the reaction liquid (excluding $Ca^{2+}$ and the drug) was gently added so as not to suspend any precipitate, and the precipitate was again removed by suction; the procedure was repeated twice and the precipitate was washed 3 times in total.

The washing solution was removed completely, and 20 μl of sample buffer for polyacrylamide gel electrophoresis (SDS-PAGE) was added and mixed well. The precipitate was suspended and heated for 5 minutes at 95°C, and then poured into a PAG MINI "No.1" 10/20 gel (Daiichi Pure Chemicals Co., Ltd) and electrophoresized for about 1 hour at 40 mA. After electrophoresis, the suspension was silver-stained using "Daiichi" 2D-silver staining reagent (Daiichi Pure Chemicals Co., Ltd), and washed with distilled water and dried. Finally, the amount of precipitate of Troponin I was calculated by scanning the suspension (EPSON ES-8500 scanner) and analyzing the resulting scan (Image J software). The results are shown in Table 7.

Table 7. Amount of precipitate of troponin I.

| Test tube No. | Condition | Ratio |
|---|---|---|
| 1 | Ca $10^{-8}$ M the compound ($10^{-5}$ M) | 1.0 |
| 2 | Ca $10^{-7}$ M the compound ($10^{-5}$ M) | 0.94 |
| 3 | Ca $10^{-6}$ M the compound ($10^{-5}$ M) | 0.81 |
| 4 | Ca $10^{-5}$ M the compound ($10^{-5}$ M) | 0.78 |

In this experiment, with a concentration of the compound in the test tube of $10^{-5}$ M the amount of troponin I which precipitated at a $10^{-8}$ M calcium concentration was set at 1.0, and the relative amount of precipitate decreased to 0.94, 0.81, and 0.78, respectively, as the calcium concentration was increased to $10^{-7}$, $10^{-6}$ and $10^{-5}$ M. This suggests that the lower the calcium concentration the greater the amount of precipitate of troponin I; that is to say, the results indicate that the amount of troponin I precipitated by the compound is influenced by the calcium concentration.

EXPERIMENT 8

Inhibition of clofilium-induced torsades de pointes by the compound

In this experiment, four white rabbits (weight: 2.8-3.2 kg) were used. Each rabbit was intravenously anesthetized with 5 mg/kg methohexital sodium. Injection catheters for the compound and test drug were inserted into the right external jugular veins of the rabbits, and microchip catheters (SPC-320, Millar) for blood pressure measurement were inserted via the right common arteries, under artificial respiration by endotracheal intubation.

The compound solution was prepared by dissolving 100 mg of the compound in 1 ml of dimethylsulfoxide (DMSO), and stored at 4°C.

A 2-lead electrocardiogram and blood pressure of each rabbit were recorded simultaneously on a personal computer via an A/D converter. In this experiment, a sequence of more than 6 polymorphic ventricular tachycardia configurations on the electrocardiogram was defined as torsades de pointes. The number of onsets of torsades de pointes was recorded by electrocardiogram for 30 minutes after administration of clofilium. Methoxamine (an α-stimulant) was used to rapidly induce torsades de pointes. The compound, methoxamine and clofilium were administered in physiological saline.

Four rabbits were used in the experiment: the first rabbit (weight: 3.0 kg) was defined as Test A, the second rabbit (weight: 3.1 kg) as Test B, the third rabbit (weight: 2.8 kg) as Control A, and the fourth rabbit (weight: 3.1 kg) as Control B.

In this experiment, methoxamine was first administered at 15 μg/kg/min, and 10 minutes later clofilium was administered for 20 minutes at 50 μg/kg/min to the rabbits defined as Tests A and B. Concomitantly with the start of clofilium administration, the compound was intravenously administered at 0.2 mg/kg/min to these rabbits. Administration of the compound was continued for 10 minutes after clofilium was discontinued. The onset of torsades de pointes in Tests A and B was continuously monitored by electrocardiogram for 10 minutes after clofilium was discontinued, i.e, for 30 minutes after clofilium was started. Compound administration and monitoring by electrocardiogram were continued during this period.

In this experiment, the study on Controls A and B was performed in the same way as the study for Tests A and B, except for compound administration. In other words, methoxamine was administered at 15 μg/kg/min, and 10 minutes later clofilium was administered for 20 minutes at 50 μg/kg/min to Controls A and B. In this experiment, the onset of torsades de pointes in Controls A and B was continuously monitored by electrocardiogram for 10 minutes after clofilium was discontinued, i.e., for 30 minutes after clofilium was started. The electrocardiogram observations are shown in Figures 1 to 5.

The electrocardiogram was monitored for 30 minutes after clofilium (50 μg/kg/min) and the compound (0.2 mg/kg/min) were intravenously administered to the rabbits defined as Tests A and B under methoxamine stimulation. However, as shown in Figure 1, the electrocardiogram for Tests A and B showed "wave 1" in which no ventricular arrhythmia was noted, and onset of torsades de pointes could not be confirmed 23 minutes after the start of continuous

intravenous injection. Also, the onset of torsades de pointes could not be confirmed 30 minutes after the start of continuous intravenous injection. These results indicate that the onset of torsades de pointes is completely prevented by the compound.

The electrocardiogram was monitored for 30 minutes after clofilium (50 μg/kg/min) was intravenously administered to Control A rabbit under methoxamine stimulation. As shown in Figure 2, "wave 3" suggested torsades de pointes was observed in the electrocardiogram of Control A 25 minutes 19 seconds after clofilium administration (indicated by arrow 2). This "wave 3" that indicated the onset of torsades de pointes stopped 25 seconds after onset, as shown in Figure 3 (indicated by arrow 4), but subsequently recurred (out of the range of Figure 3, not shown in the figure).

The electrocardiogram was monitored for 30 minutes after clofilium (50 μg/kg/min) was intravenously administered to Control B rabbit under methoxamine stimulation. As shown in Figure 4, "wave 3" suggesting torsades de pointes was observed in the electrocardiogram of Control B 22 minutes 30 seconds after clofilium administration (indicated by arrow 5). This "wave 3" that indicated the onset of torsades de pointes stopped 49 seconds after onset as shown in Figure 5 (indicated by arrow 6), but subsequently recurred (out of the range of Figure 5, not shown in the figure). These electrocardiogram observations are shown in Table 8.

Table 8

| Rabbit No. | Test drug | Incidence of torsades de pointes |
|---|---|---|
| Test A | clofilium + methoxamine + the compound | 0 |
| Test B | clofilium + methoxamine + the compound | 0 |
| Control A | clofilium + methoxamine | 9 |
| Control B | clofilium + methoxamine | 4 |

Torsades de pointes was induced by clofilium in Controls A and B, but torsades de pointes was not induced by clofilium combined with the compound in Tests A and B. This indicates that the compound completely inhibited torsades de pointes, which otherwise recurred repeatedly after clofilium administration. In addition, torsades de pointes induced by drugs or electrolyte abnormalities disappears with elimination of the cause. It means that the cause of torsades de pointes is eliminated when administering the compound in this experiment. Since the compound is able to stop arrhythmia without inducing torsades de pointes, treatment of arrhythmia associated with torsades de pointes can be carried out while inhibiting the onset of torsades de pointes, or by eliminating the cause of torsades de pointes when the compound is administered.

Clofilium has been used in many experiments to confirm onset of torsades de pointes, and therefore it was chosen for use in this experiment. As alternatives instead of clofilium, the following can be used:

antiarrhythmic agents for treatment of arrhythmia which causes delayed repolarization, including disopyramide, quinidine, procainamide and
propafenone, which are classified as Class IA drugs in the Vaughan Williams classification of antiarrhythmic agents and have an inhibitory effect on sodium channels; or amiodarone and nifekalant hydrochloride, which are classified as Class III drugs in the Vaughan Williams classification.

EXPERIMENT 9

Effect of the compound on blood pressure

Effects of the compound on blood pressure were examined in the same way as described in Experiment 2. In this experiment, Control and Tests 6-9 were anesthetized with a single intravenous injection of 20 mg/kg methohexital sodium under artificial respiration (Type AR1 Narishige Group) using endotracheal intubation. The 1,4-benzothiazepine derivative or pharmaceutically acceptable salt thereof was used as the compound in this experiment.

Five white rabbits (weight: 2.7 - 2.9 kg) were used in this experiment: a rabbit (weight: 2.8 kg, Control) that received control solution, a rabbit (weight: 2.7 kg, Test 6) that received the compound at 0.04 mg/kg/min, a rabbit (weight: 2.7 kg, Test 7) that received the compound at 0.04 mg/kg/min, a rabbit (weight: 2.9 kg, Test 8) that received the compound at 0.4 mg/kg/min, and a rabbit (weight: 2.8 kg, Test 9) that received the compound at 0.4 mg/kg/min.

Each rabbit was anesthetized with a single intravenous injection of 20 mg/kg methohexital sodium under artificial respiration (Type AR1 Narishige Group) using endotracheal intubation. Subsequently, α-chloralose was injected into the ear veins at 90 mg/kg/20min. A DMSO solution of the compound was prepared by dissolving 100 mg of the compound in 1 ml of dimethylsulfoxide (DMSO), and stored at 4°C.

In this experiment, injection catheters for the compound for administration of drugs were inserted into the right external jugular veins of the rabbits and microchip catheters (SPC-320, Millar) for blood pressure measurement were

inserted via the right common arteries. A 2-lead electrocardiogram was examined and blood pressure, cardiac rate, and electrocardiogram of the rabbits were monitored for 10 minutes. When the data stabilized, the electrocardiogram, systolic pressure and diastolic pressure of each rabbit were recorded on a personal computer via an A/D converter. The Control rabbit was administered 5% dextrose solution containing 0.1% DMSO as a control solution for 10 minutes at 0.1 ml/min. Test 6 and 7 rabbits were administered 5% dextrose containing the compound at 0.1 ml/min, and then the compound at an infusion rate of 0.04 mg/kg/min. Test 8 and 9 rabbits were administered 5% dextrose containing the compound at 0.1 ml/min, and then the compound at an infusion rate of 0.4 mg/kg/min.

Systolic pressure (mmHg) and diastolic pressure (mmHg) of the Control and Test rabbits were measured over 5 heart beats before administration of the solution, and 5 and 10 minutes after administration. The mean blood pressure (mmHg) was calculated from the systolic pressure (mmHg) and the diastolic pressure (mmHg), using the formula: mean blood pressure (mmHg) = [(systolic pressure + diastolic pressure) $\times$ 1/2]. The results are shown in Table 9.

Table 9

Effects of the infusion rate of the compound (mg/kg/min) on mean blood pressure (mmHg)

| Infusion rate | 0 | 0.04 | 0.04 | 0.4 | 0.4 |
|---|---|---|---|---|---|
| (Measurement time) | Control | Test 6 | Test 7 | Test 8 | Test 9 |
| Before administration | 78.3 (100%) | 85.6 (100%) | 87.7 (100%) | 74.0 (100%) | 97.7 (100%) |
| After 5 minutes | 75.4 (96.3%) | 79.5 (92.9%) | 82.0 (93.5%) | 35.7 (48.2%) | 64.5 (66.0%) |
| After 10 minutes | 78.7 (100.5%) | 85.9 (100.4%) | 93.4 (106.5%) | 41.7 (56.4%) | 46.3 (47.4%) |

The mean blood pressure of the Control 10 minutes after administration of the control solution was 100.5%, where each mean blood pressure before administration was defined as 100%, and there were no significant differences in measured parameters before and after administration of the control solution.

The mean blood pressures of Test 6 and 7 rabbits, which were administered the compound at 0.04 mg/kg/min, were 100.4% and 106.5%, respectively, 10 minutes after administration, where each mean blood pressure before the administration was defined as 100%; these values were similar to that of the normal Control, and there were no significant differences in mean blood pressure among these animals. In the Test 8 rabbit, which was administered the compound at 0.4 mg/kg/min, the mean blood pressure was 48.2% and 56.4% 5 and 10 minutes after administration, respectively, where the mean blood pressure before administration was defined as 100%; therefore, the mean blood pressure decreased by more than 40% compared to the value before administration. In the Test 9 rabbit, which was administered the compound at 0.4 mg/kg/min, the mean blood pressure was 66.0% and 47.4% 5 and 10 minutes after administration, respectively; therefore, the mean blood pressure decreased by more than 50% compared to the value before administration. In conclusion, the results indicate that the compound has an effect of decreasing blood pressure in a concentration-dependent fashion, and therefore may be used as a therapeutic agent for hypertension.

EXPERIMENT 10

Effect of the compound on cardiac rate and PQ interval on the electrocardiogram (conduction velocity through the excitation-conduction system between the atrium and the ventricle)

In this experiment, the 1-hydrochloride of the compound, 4-[3-(4-benzylpiperidin-1-yl)propionyl]-7-methoxy-2,3,4,5-tetrahydro-1,4-be nzothiazepine 1-hydrochloride (hereinafter called the compound) was used as the 1,4-benzothiazepine derivative or pharmaceutically acceptable salt thereof.

Five white rabbits (weight: 2.7-2.9 kg) were used in this experiment: a rabbit (weight 2.8 kg, Control 1) that was administered control solution, a rabbit (weight 2.7 kg, Test 10) that received the compound at 0.04 mg/kg/min, a rabbit (weight 2.7 kg, Test 11) that received the compound at 0.04 mg/kg/min, a rabbit (weight 2.9 kg, Test 12) that received the compound at 0.4 mg/kg/min, and a rabbit (weight 2.8 kg, Test 13) that received the compound at 0.4 mg/kg/min.

Each rabbit was anesthetized with a single intravenous injection of 20 mg/kg methohexital sodium under artificial respiration (Type AR1 Narishige Group) using endotracheal intubation. Subsequently, $\alpha$-chloralose was injected into the ear veins at 90 mg/kg/20min. A DMSO solution of the compound was prepared by dissolving 100 mg of the compound in 1 ml of dimethylsulfoxide (DMSO), and stored at 4°C.

In this experiment, injection catheters for administration of the compound and other drugs were inserted into the right external jugular veins of the rabbits, and microchip catheters (SPC-320, Millar) for blood pressure measurement were inserted via the right common arteries. A 2-lead electrocardiogram was examined, and blood pressure, cardiac

rate, and electrocardiogram of the rabbits were monitored for 10 minutes. When the data stabilized, the electrocardiogram, systolic pressure and diastolic pressure of each rabbit were recorded on a personal computer via an A/D converter. The Control rabbit was administered 5% dextrose solution containing 0.1 % DMSO as a control solution for 10 minutes at 0.1 ml/min. Test 10 and 11 rabbits were administered 5% dextrose containing the compound at 0.1 ml/min, and then the compound at an infusion rate of 0.04 mg/kg/min. Test 12 and 13 were administered 5% dextrose containing the compound at 0.1 ml/min, and then the compound at an infusion rate of 0.4 mg/kg/min.

Cardiac rate and PQ interval in the Control and Test rabbits were measured before administration of the test solution, and 5 and 10 minutes after administration. The PQ interval, which is the time interval between the beginning of the P-wave and the beginning of next Q wave, reflects the conduction velocity of electrical stimulation from the atrium to the ventricle. The cardiac rates before administration of the test solution were defined as 100%, and data for the cardiac rate after administration of the compound are shown in Table 10.

Table 10

Cardiac rate/min for different infusion rates (mg/kg/min) of the test compound

| Infusion rate | 0 | 0.04 | 0.04 | 0.4 | 0.4 |
|---|---|---|---|---|---|
| (Measurement time) | Control | Test 10 | Test 11 | Test 12 | Test 13 |
| Before administration | 334 (100%) | 297 (100%) | 286 (100%) | 372 (100%) | 327 (100%) |
| After 5 minutes | 332 (99.4%) | 289 (97.3%) | 283 (99.0%) | 350 (94.1%) | 311 (95.1%) |
| After 10 minutes | 328 (98.2%) | 286 (96.3%) | 281 (98.3%) | 157 (42.2%) | 98 (30.0%) |

Each cardiac rate before administration of the control solution or the compound was defined as 100%. The cardiac rate in the Control rabbit, which was not administered the compound, was 98.2% 10 minutes after commencement of the measurement, and there were no significant differences in cardiac rate before and after administration of the control solution.

The cardiac rates of Test 10 and 11 rabbits, which were administered the compound at 0.04 mg/kg/min, were 96.3% and 98.3%, respectively, 10 minutes after administration, where the cardiac rate before administration of the compound was defined as 100%; these values were similar to that of the Control, and there were no significant differences in the cardiac rate among these animals. In Test 12 and 13 rabbits, the cardiac rate before administration was defined as 100%. These rabbits were administered the compound at 0.4 mg/kg/min, and the cardiac rates of Test 12 were 94.1% and 42.2% 5 and 10 minutes after administration, respectively; therefore, the cardiac rate decreased by more than 55% compared to before administration. Similarly, the cardiac rates of Test 13 were 95.1% and 30.0% 5 and 10 minutes after administration, respectively; therefore, the cardiac rate decreased by 70% compared to before administration.

Table 11

PQ interval (milliseconds) for infusion rates of the compound (mg/kg/min)

| Infusion rate | 0 | 0.04 | 0.04 | 0.4 | 0.4 |
|---|---|---|---|---|---|
| (Measurement time) | Control | Test 10 | Test 11 | Test 12 | Test 13 |
| Before administration | 64 (100%) | 71 (100%) | 64 (100%) | 69 (100%) | 68 (100%) |
| After 5 minutes | 65 (101.6%) | 73 (102.8%) | 66 (103.1 %) | 92 (133.3%) | 82 (120.6%) |
| After 10 minutes | 64 (100%) | 74 (104.2%) | 68 (106.3%) | 107 (155.1 %) | 103 (151.5%) |

Each PQ interval before administration of the control solution was defined as 100%. The PQ interval of the Control rabbit, which was not administered the compound, was 100% 10 minutes after commencement of the measurement, and there was no difference between before and after administration of the control solution. The PQ intervals for the Test 10 and 11 rabbits, which were administered the compound at 0.04 mg/kg/min, were 104.2% and 106.3%, respectively, 10 minutes after administration, where the PQ interval before administration of the control drug was defined as 100%; therefore, there were no significant differences in the PQ interval, similarly to the Control. In the Test 12 and 13 rabbits, which were administered the compound at 0.4 mg/kg/min, the PQ interval before administration was defined as 100%. The PQ intervals of the Test 12 rabbit were 133.3% and 155.1 % 5 and 10 minutes after administration,

respectively; therefore, the PQ interval increased by approximately 50% compared to before administration. Furthermore, the PQ intervals of the Test 13 rabbit were 120.6% and 151.5% 5 and 10 minutes after administration, respectively; therefore, the PQ interval increased by approximately 50% compared to before administration. In conclusion, the results indicate that the compound has an effect of prolonging the PQ interval in a concentration-dependent manner, and therefore may be used as a therapeutic agent for sinus tachycardia and supraventricular tachycardia.

EXPERIMENT 11

Examination of the protective effect of the compound on diastolic dysfunction of the myocardium

This experiment was performed to examine the protective effect of the compound on left ventricular diastolic dysfunction, and the compound was administered 5 minutes before administration of the norepinephrine solution referred to in Experiment 1, in which a calcium solution and a norepinephrine solution were administered after calcium administration for 20 minutes.

In this experiment, the 1,4-benzothiazepine derivative or pharmaceutically acceptable salt thereof was used as the compound.

Eight-week old male Wistar rats weighing 310-320 g were used in the study. The rats were anesthetized with 1,000 mg/kg of urethane and 80 mg/kg of $\alpha$-chloralose by subperitoneal injection under artificial respiration (SN-480-7, Shinano) using endotracheal intubation. In this experiment, 100 mg of the compound was dissolved in 1 ml of dimethylsulfoxide (DMSO) and the resulting DMSO solution of the compound was stored at 4°C. Norepinephrine solution was prepared by dissolving 1 mg of norepinephrine in 41 $\mu$l of distilled water, at an infusion speed of 40 $\mu$g/kg/min.

Firstly, continuous infusion catheters of calcium chloride solution or norepinephrine solution containing calcium chloride were inserted into the right external jugular veins of the rats, microchip catheters (SPC-320, Millar) was inserted into the left ventricles via the right common arteries, and the left ventricular end-diastolic pressure was measured. In addition, test drug infusion catheters were inserted into the right femoral veins.

A 1-lead electrocardiogram and the left ventricular pressure were recorded simultaneously on a personal computer via an A/D converter. The left ventricular diastolic pressure was determined as the mean of data measured over 20 heart beats every 5 minutes, when the pressure equivalent to the R wave of the electrocardiogram was defined as the left ventricular diastolic pressure. In the preparation step, blood pressure, pulse, and electrocardiogram of the rats were monitored for 15 minutes and allowed to stabilize, and then 5% dextrose solution containing calcium chloride (at a concentration based on the weight of the rat) was infused into the right external jugular vein for 20 minutes at 16.6 $\mu$l/min (12.0 mg/kg/min of calcium chloride).

Secondly, a norepinephrine solution containing calcium chloride without a change in dosage was immediately injected at a rate of 30 $\mu$g/kg/min via the right external jugular vein. After the start of administration, calcium chloride and norepinephrine were then continuously injected in the form of a norepinephrine solution containing calcium chloride. With continuous injection of calcium chloride and norepinephrine (in the form of a norepinephrine solution containing calcium chloride) via the right external jugular vein, 0.2 ml of 1% DMSO solution of the solvent used for the test compound was administered over 30 seconds to a rat (weight: 310 g, Control; this animal was not administered the compound) via the right femoral vein, 5 minutes before commencement of injection (intravenous injection) of calcium chloride and norepinephrine (in the form of a norepinephrine solution containing calcium chloride) via the right external jugular vein.

Similarly to the Control, with continuous injection of calcium chloride and norepinephrine (in the form of a norepinephrine solution containing calcium chloride) via the right external jugular vein, 0.2 ml of a 1% DMSO solution containing 0.3 mg/kg of the compound was administered over 30 seconds to a rat (weight: 320 g, defined as Test 14) via the right femoral vein, 5 minutes before commencement of injection (intravenous injection) via the right external jugular vein of calcium chloride and norepinephrine. Furthermore, in this experiment, after the test drug was injected over 30 seconds, a 1:10 dilution of the injected test drug was additionally administered at 10 $\mu$l/min from commencement of test drug injection to 15 minutes after the start of administration. In the Test 14 animal, after 0.2 ml of 1% DMSO solution of the compound containing 0.3 mg/kg of such test drug was injected over 30 seconds, a 1% DMSO solution of the compound was additionally injected at 0.02 mg/kg/min from the commencement of test drug injection to 15 minutes after the start of administration. In this experiment, all test drugs were dissolved in 5% dextrose solution. The left ventricular diastolic pressure of each rat was determined as the mean of data measured over 20 heart beats every 5 minutes, when the pressure equivalent to the R wave of the electrocardiogram was defined as the left ventricular diastolic pressure. The experiment was completed 15 minutes after commencement of the injection of the compound or control solution.

Furthermore, the left ventricular diastolic function of each rat was examined using tissue Doppler ultrasonography. Ultrasonic diagnostic equipment (Toshiba Powervision SSA-380APSK-70LT, Toshiba Corporation) was used and the rats were examined using ultrasound at 10 MHz. Firstly, the precordial region of each rat was shaved, and an ECHO probe was placed on the cardiac apex. The long axis of the left ventricle of the cardiac apex was imaged, a sample volume for pulsed-wave Doppler was established at the base of the posterior mitral leaflet, and the velocity of the left ventricular posterior wall motion [Ea wave (m/sec)] was measured at diastole as the left ventricular diastolic

function. The left ventricular end-diastolic pressure and the velocity of the left ventricular posterior wall motion [Ea wave (m/sec)] in the Control and Test 14 animals, which were administered a control solution or the compound 5 minutes before measurement, are shown in Table 12. In this experiment, the left ventricular diastolic function is defined as the ratio of the velocity of the left ventricular wall motion at diastole before intravenous injection of norepinephrine (before administration; i.e. a normal left ventricular wall) determined using tissue Doppler imaging, or the ratio of the velocity of the left ventricular wall motion at diastole after intravenous injection of norepinephrine (after administration). The results are shown in Table 12.

Table 12

| Elapsed time | left ventricular end-diastolic pressure | |
|---|---|---|
| | Control | Test 14 |
| 25 minutes before<br>(5 minutes before the start of $CaCl_2$ administration) | 7.4 | 7.2 |
| 20 minutes before<br>(just after the start of $CaCl_2$ administration) | 7.6 | 7.7 |
| 10 minutes before<br>(10 minutes after the start of $CaCl_2$ administration) | 8.2 | 7.9 |
| Just before<br>(20 minutes after the start of $CaCl_2$ administration) | 8.0 | 8.1 |
| 5 minutes after<br>(5 minutes after the start of test drug administration)<br>(just before the start of norepinephrine intravenous injection) | 8.2 | 8.0 |
| 10 minutes after<br>(10 minutes after the start of test drug administration)<br>(5 minutes after the start of norepinephrine intravenous injection) | 28.8 | 14.5 |
| 15 minutes after<br>(15 minutes after the start of test drug administration)<br>(10 minutes after the start of norepinephrine intravenous injection) | 46.4 | 18.3 |

In the Control, the left ventricular end-diastolic pressure before the start of calcium chloride administration was 7.4 mmHg and the pressure was 8.0 mmHg just before the start of administration of the control solution. The left ventricular end-diastolic pressure 5 minutes after the start of norepinephrine intravenous injection was 28.8 mmHg; the pressure increased to 46.4 mmHg 10 minutes after commencement of norepinephrine intravenous injection.

In the Test 14 animal, the left ventricular end-diastolic pressure before the start of calcium chloride administration was 7.2 mmHg, and the pressure was 8.1 mmHg just before the start of administration of the compound. It was 14.5 mmHg 5 minutes after the start of norepinephrine administration and 18.3 mmHg 10 minutes after the start of norepinephrine administration. Compared to the Control, it is evident that administration of the compound can suppress elevation of the left ventricular end-diastolic pressure. The same results were obtained when the compound was administered before administration of a norepinephrine solution containing calcium chloride, showing that the compound can inhibit an increase in left ventricular end-diastolic pressure and improve diastolic dysfunction.

EXPERIMENT 12

Effect of the compound on myocardial microcirculation

The effects of the compound on myocardial microcirculation were examined. In this experiment, the 1-hydrochloride of the compound, 4-[3-(4-benzylpiperidin-1-yl)propionyl]-7-methoxy-2,3,4,5-tetrahydro-1,4-be nzothiazepine 1-hydrochloride (hereinafter called the compound) was used as the 1,4-benzothiazepine derivative or pharmaceutically acceptable salt thereof.

Four 8-week old male Wistar rats weighing 300-310 g were used in the study. The rats were anesthetized with 1,000 mg/kg of urethane and 80 mg/kg of α-chloralose by subperitoneal injection under artificial respiration (Harvard) using endotracheal intubation. In this experiment, 100 mg of the compound was dissolved in 1 ml of dimethylsulfoxide (DMSO) and the resulting DMSO solution of the compound was stored at 4°C. Norepinephrine solution was prepared by dissolving 1 mg of norepinephrine in 41 μl of distilled water, at an infusion speed of 30 μg/kg/min.

Firstly, continuous infusion catheters of calcium chloride solution or norepinephrine solution containing calcium chloride were inserted into the right external jugular veins of the rats, and 2F polyethylene catheters for microspheres were inserted into the left ventricles via the right common arteries. In addition, test drug infusion catheters were inserted

into the right femoral veins.

A 1-lead electrocardiogram and the left ventricular pressure were recorded simultaneously on a personal computer via an A/D converter. In the preparation step, blood pressure, pulse, and electrocardiogram of the rats were monitored for 15 minutes and allowed to stabilize, and then 5% dextrose solution containing calcium chloride (at a concentration based on the weight of the rat) was infused into the right external jugular vein for 20 minutes at 16.6 μl/min (12 mg/kg/min of calcium chloride). Next, norepinephrine solution containing calcium chloride without changes in dosage regimen was immediately injected at a rate of 30 μg/kg/min via the right external jugular vein. After the start of administration, calcium chloride and norepinephrine were then continuously injected in the form of a norepinephrine solution containing calcium chloride. With continuous injection of calcium chloride via the right external jugular vein, 5% dextrose solution containing 0.1 % DMSO solution was administered via the right femoral vein to Control A rat weighing 310 g (to which the compound was not administered) and to Control B rat weighing 300 g (to which the compound was not administered), 5 minutes before commencement of injection (intravenous injection) of the norepinephrine solution containing calcium chloride. In addition, with continuous injection of calcium chloride via the right external jugular vein, a 5% dextrose solution of the compound was continuously administered to Test 15 rat weighing 300 g and Test 16 rat weighing 300g for 3 minutes at a rate of 0.33 mg/kg/min and subsequently for 25 minutes at a rate of 0.01 mg/kg/min, 5 minutes before commencement of injection (intravenous injection) of the norepinephrine solution containing calcium chloride via the right external jugular vein. The infusion rate was 0.5 ml/3 minutes for the first 3 minutes and then 0.05 ml/3 minutes.

Myocardial tissue blood flow rate was measured twice using microspheres, 5 minutes before the start of calcium chloride administration and 20 minutes after the start of administration of norepinephrine solution containing calcium chloride. Approximately 200,000 microspheres/rat [1: Yellow DYE-TRAK) VII+, 2: Persimmon DYE-TRAK VII+ (TRITON Technologies, Inc.)] were injected over 50 seconds at 0.6ml/min using an infusion pump (Model KDS230) via the polyethylene catheter placed in the left ventricle, while reference blood samples were collected via catheters placed in the femoral artery. The samples were aspirated and collected at 0.84 ml/min using an infusion pump (Model KDS230) over 75 seconds from 10 seconds before microsphere infusion. After completion of measurements, left ventricles were isolated and weighed. Pigment was extracted by dissolving the left ventricle and the reference blood samples, and absorbance of the pigment was measured using a double beam spectrophotometer (150-20, Hitachi Ltd.). Blood flow rate in the tissues was calculated from the amount of microspheres measured in the tissues and in the reference blood samples.

The local blood flow rate was calculated using the following formula.

$$Qm = (Am \times Qr)/Ar \text{..........formula}$$

Where Qm is the blood flow rate of the sample (ml/min/g (myocardium)), Qr is the collection rate for the reference blood samples (ml/min), Am is the absorbance of microspheres in 1g of the tissues, and Ar is the absorbance of all microspheres in the reference blood samples. The results for the myocardial tissue blood flow rate are shown in Table 13.

Table 13

| Experiment | Myocardial tissue blood flow rate (ml/min/g) and ratio (%) | |
|---|---|---|
| | 5 minutes before the start of $CaCl_2$ administration | 20 minutes after the start of norepinephrine solution containing $CaCl_2$ |
| Control A (solvent) | 5.5 (100%) | 2.2 (41%) |
| Control B (solvent) | 5.1 (100%) | 2.9 (57%) |
| Test 15 (the compound) | 4.6 (100%) | 3.2 (70%) |
| Test 16 (the compound) | 4.3 (100%) | 4.2 (98%) |

In Controls, the myocardial tissue blood flow rate 20 minutes after the start of administration of norepinephrine containing calcium chloride was 41 % in Control A and 57% in Control B, where the myocardial tissue blood flow rate 5 minutes before the start of calcium chloride administration was defined as 100%. In the experiments in which the compound was administered, the myocardial tissue blood flow rate 20 minutes after the start of administration of norepinephrine containing calcium chloride was 70% in Test 15 and 98% in Test 16, where the myocardial tissue blood flow rate 5 minutes before the start of calcium chloride administration was defined as 100%. The results show that the myocardial tissue blood flow rate decreased to approximately 41% and 57%, respectively, in Controls A and B, in which the solvent without the compound was administered, while decreases in the myocardial tissue blood flow rate were

prevented in the Test 15 and 16 animals, in which the compound was administered.

In general, myocardial tissue blood flow decreases by approximately 50% when diastolic dysfunction develops, but the decrease in blood flow can be suppressed to 30% or less by administration of the compound. The result indicates that the compound improves diastolic dysfunction of the myocardium, increases the blood flow to myocardial tissues, and improves impaired myocardial microcirculation, because blood principally enters into myocardial tissues at diastole.

INDUSTRIAL APPLICABILITY

A drug containing the 1,4-benzothiazepine derivative or pharmaceutically acceptable salt thereof as an active ingredient has activity to relax muscles such as the myocardium, skeletal muscles, and smooth muscles. For example, the drug can relax the cardiac muscle without affecting myocardial contraction in a short time or within a desirable time after administration. This can improve blood flow, for example, in myocardial coronary circulation, and especially in myocardial microcirculation; therefore, cardiomegaly, and particularly severe aortic stenosis, and angina pectoris associated with aortic incompetence, can be treated. Furthermore, for example, hypertensive cardiac diseases, idiopathic hypertrophic cardiomyopathy, and myocardial damage showing depression of ST segment on the electrocardiogram can be treated and prevented by accelerating relaxation of cardiac muscle with administration of the drug. Furthermore, for example, myocardial relaxation failure, such as failure of left ventricular dilation can be treated and prevented by accelerating relaxation of cardiac muscle with administration of the drug. Furthermore, for example, the drug can treat acute pulmonary edema caused by intractable left ventricular diastolic dysfunction. In addition, the drug can serve as a therapeutic agent for hypertension, especially, catecholamine-induced hypertension, by relaxing the peripheral vessel, and also can serve as a therapeutic agent for frequent arrhythmia with a short diastolic phase, especially in ventricular tachycardia. Furthermore, the agent can serve as a prophylactic agent and a therapeutic agent for drug-induced torsades de pointes which is no existence until now, and this is a good thing for patients with torsades de pointes.

In conclusion, the drug containing the 1,4-benzothiazepine derivative or pharmaceutically acceptable salt thereof as an active ingredient can serve as a therapeutic agent and a prophylactic agent for new many diseases. The drug will be extremely useful in treating, effects brought to society by the drug will be significant, and also industrial applicability of the drug will be significant.

## Claims

1. An accelerating agent for muscular relaxation comprising 1,4-benzothiazepine derivatives represented by the following general formula [I] or a pharmaceutically acceptable salt thereof as an active ingredient;

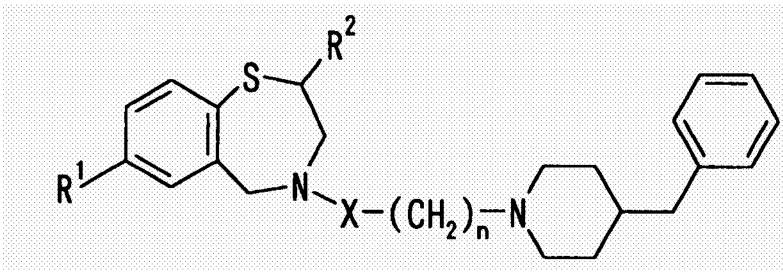

[I]

where $R^1$ represents a hydrogen atom or C1-C3 lower alkoxy group; $R^2$ represents a hydrogen atom, C1-C3 lower alkoxy group or phenyl group, wherein said phenyl group may be substituted with 1-3 substituents selected from the group consisting of a hydroxyl group and a C1-C3 lower alkoxy group, or a group represented by the following formula,

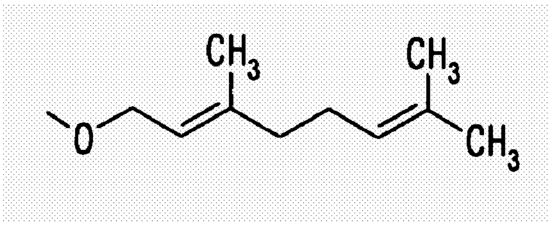

or

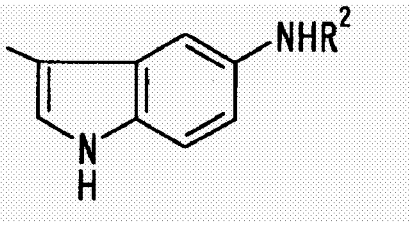

where $R^3$ represents a C1-C3 acyl group; X represents -CO- or $-CH_2-$; and n represents 1 or 2.

**2.** An accelerating agent for muscular relaxation comprising 1,4-benzothiazepine derivatives represented by the following general formula [I] or a pharmaceutically acceptable salt thereof as an active ingredient and having an effect of enhancing binding strength to actin-tropomyosin complex of troponin I of a protein inhibiting muscle contraction in muscle;

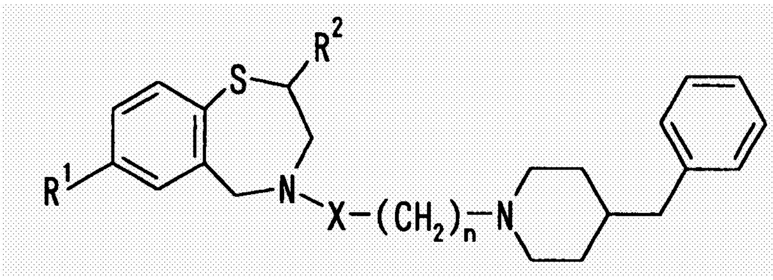

[I]

where $R^1$ represents a hydrogen atom or C1-C3 lower alkoxy group; $R^2$ represents a hydrogen atom, C1-C3 lower alkoxy group or phenyl group, wherein said phenyl group may be substituted with 1-3 substituents selected from the group consisting of a hydroxyl group and a C1-C3 lower alkoxy group, or a group represented by the following formula,

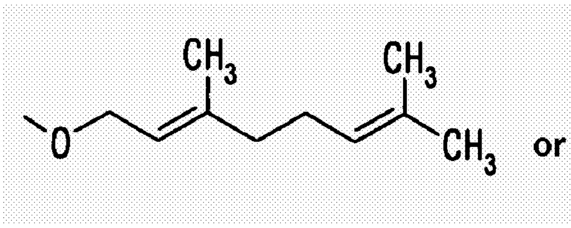

or

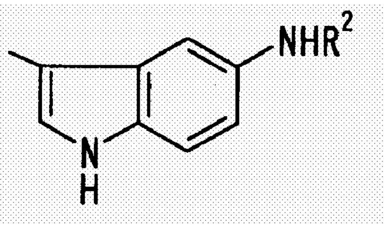

where $R^3$ represents a C1-C3 acyl group; X represents -CO- or $-CH_2-$; and n represents 1 or 2.

**3.** A An accelerating agent for muscular relaxation comprising a 1,4-benzothiazepine derivatives represented by the general formula [1] or a pharmaceutically acceptable salt thereof as defined in claim 1 or 2, wherein said 1,4-benzothiazepine derivatives is 4-[3- (4-benzylpiperidine-1-yl) propionyl]-7-methoxy-2,3,4,5 -tetrahydro- 1,4- benzothiazepine, or a pharmaceutically acceptable salt thereof.

**4.** An accelerating agent for muscular relaxation as defined in claim 1 or 2, wherein the muscle is skeletal muscle.

**5.** An accelerating agent for muscular relaxation as defined in claim 1 or 2, wherein the muscle is smooth muscle.

**6.** An accelerating agent for muscular relaxation as defined in claim 1 or 2, wherein the muscle is cardiac muscle.

**7.** An accelerating agent for muscular relaxation as defined in claim 1 or 2, wherein the muscle is left ventricle muscle.

**8.** A therapeutic agent for left ventricular diastolic dysfunction comprising 1,4-benzothiazepine derivatives represented by the following general formula [I] or a pharmaceutically acceptable salt thereof as an active ingredient;

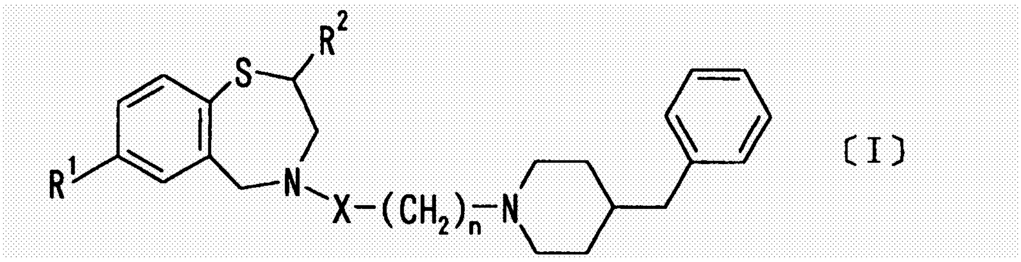

where $R^1$ represents a hydrogen atom or C1-C3 lower alkoxy group; $R^2$ represents a hydrogen atom, C1-C3 lower alkoxy group or phenyl group, wherein said phenyl group may be substituted with 1-3 substituents selected from the group consisting of a hydroxyl group and a C1-C3 lower alkoxy group, or a group represented by the following formula,

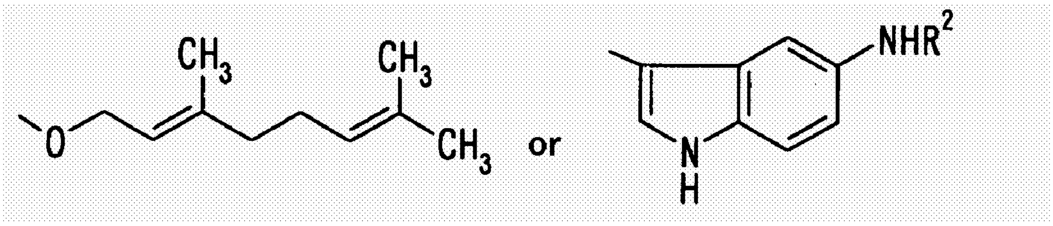

where $R^3$ represents a C1-C3 acyl group; X represents -CO- or $-CH_2-$; and n represents 1 or 2.

**9.** A therapeutic agent for left ventricular diastolic dysfunction comprising 1,4-benzothiazepine derivatives represented by the following general formula [I] or a pharmaceutically acceptable salt thereof as an active ingredient and having an effect of enhancing binding strength to actin-tropomyosin complex of troponin I of a protein inhibiting muscle contraction in muscle;

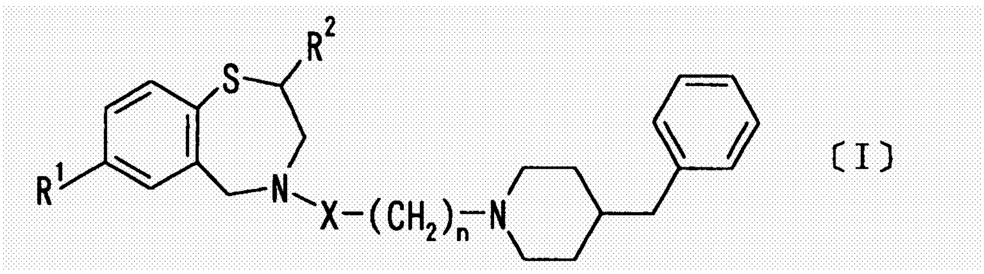

where $R^1$ represents a hydrogen atom or C1-C3 lower alkoxy group; $R^2$ represents a hydrogen atom, C1-C3 lower alkoxy group or phenyl group, wherein said phenyl group may be substituted with 1-3 substituents selected from the group consisting of a hydroxyl group and a C1-C3 lower alkoxy group, or a group represented by the following formula,

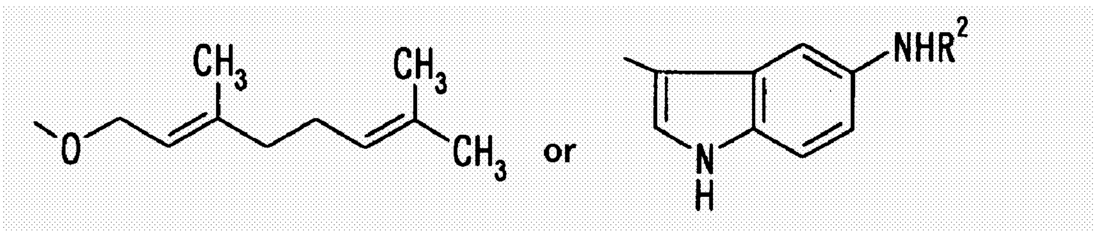

where $R^3$ represents a C1-C3 acyl group; X represents -CO- or $-CH_2-$; and n represents 1 or 2.

**10.** A therapeutic agent for left ventricular diastolic dysfunction comprising a 1,4-benzothiazepine derivatives represented

by the general formula [1] or a pharmaceutically acceptable salt thereof as defined in claim 8 or 9, wherein said 1,4-benzothiazepine derivatives is 4-[3- (4-benzylpiperidine-1-yl) propionyl]-7-methoxy-2,3,4,5 -tetrahydro- 1,4- benzothiazepine, or a pharmaceutically acceptable salt thereof.

**11.** A therapeutic agent for heart failure comprising 1,4-benzothiazepine derivatives represented by the following general formula [I] or a pharmaceutically acceptable salt thereof as an active ingredient;

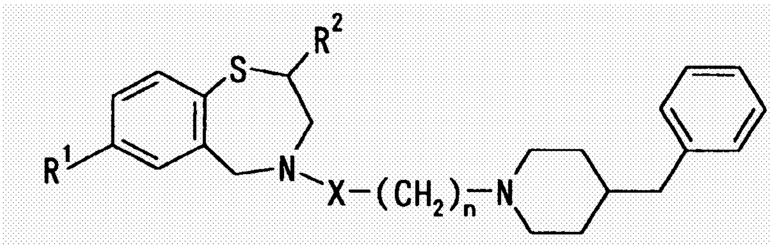

[I]

where $R^1$ represents a hydrogen atom or C1-C3 lower alkoxy group; $R^2$ represents a hydrogen atom, C1-C3 lower alkoxy group or phenyl group, wherein said phenyl group may be substituted with 1-3 substituents selected from the group consisting of a hydroxyl group and a C1-C3 lower alkoxy group, or a group represented by the following formula,

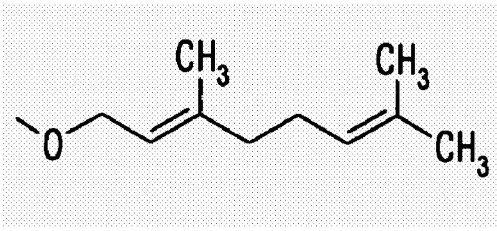

or

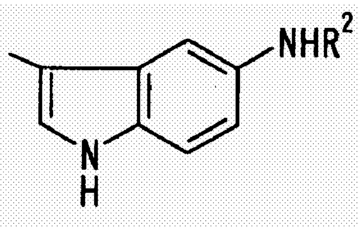

where $R^3$ represents a C1-C3 acyl group; X represents -CO- or $-CH_2-$; and n represents 1 or 2.

**12.** A therapeutic agent for heart failure comprising 1,4-benzothiazepine derivatives represented by the following general formula [I] or a pharmaceutically acceptable salt thereof as an active ingredient and having an effect of enhancing binding strength to actin-tropomyosin complex of troponin I of a protein inhibiting muscle contraction in muscle;

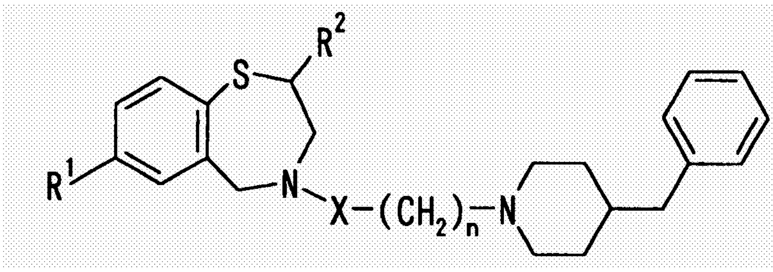

[I]

where $R^1$ represents a hydrogen atom or C1-C3 lower alkoxy group; $R^2$ represents a hydrogen atom, C1-C3 lower alkoxy group or phenyl group, wherein said phenyl group may be substituted with 1-3 substituents selected from the group consisting of a hydroxyl group and a C1-C3 lower alkoxy group, or a group represented by the following formula,

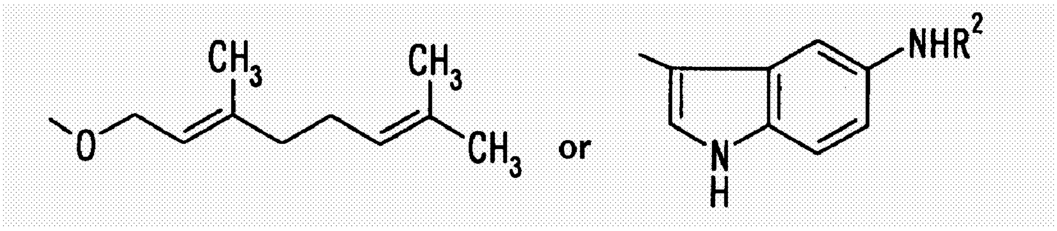

where $R^3$ represents a C1-C3 acyl group; X represents -CO- or $-CH_2-$; and n represents 1 or 2.

**13.** A therapeutic agent for heart failure comprising a 1,4-benzothiazepine derivatives represented by the general formula [1] or a pharmaceutically acceptable salt thereof as defined in claim 11 or 12, wherein said 1,4-benzothiazepine derivatives is 4-[3- (4-benzylpiperidine-1-yl) propionyl]-7-methoxy-2,3,4,5 -tetrahydro- 1,4- benzothiazepine, or a pharmaceutically acceptable salt thereof.

**14.** A therapeutic agent for acute pulmonary edema comprising 1,4-benzothiazepine derivatives represented by the following general formula [I] or a pharmaceutically acceptable salt thereof as an active ingredient;

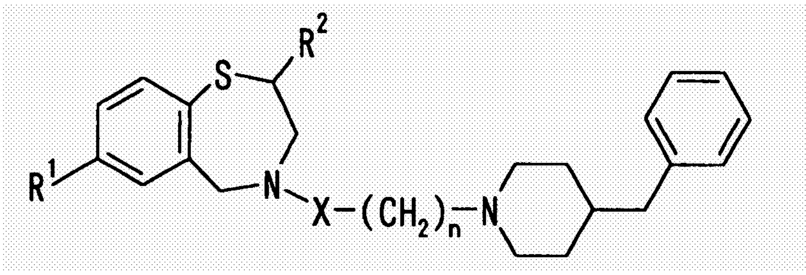

[I]

where $R^1$ represents a hydrogen atom or C1-C3 lower alkoxy group; $R^2$ represents a hydrogen atom, C1-C3 lower alkoxy group or phenyl group, wherein said phenyl group may be substituted with 1-3 substituents selected from the group consisting of a hydroxyl group and a C1-C3 lower alkoxy group, or a group represented by the following formula,

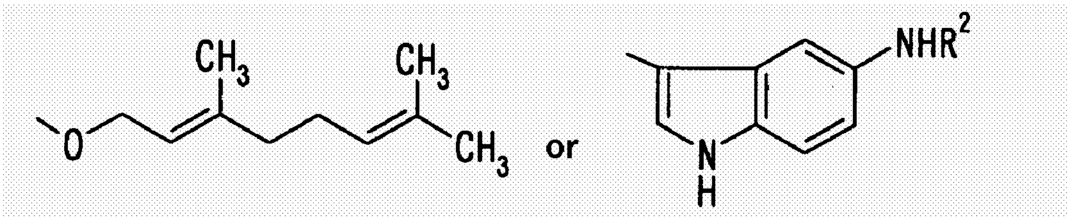

where $R^3$ represents a C1-C3 acyl group; X represents -CO- or $-CH_2-$; and n represents 1 or 2.

**15.** A therapeutic agent for acute pulmonary edema comprising 1,4-benzothiazepine derivatives represented by the following general formula [I] or a pharmaceutically acceptable salt thereof as an active ingredient and having an effect of enhancing binding strength to actin-tropomyosin complex of troponin I of a protein inhibiting muscle contraction in muscle;

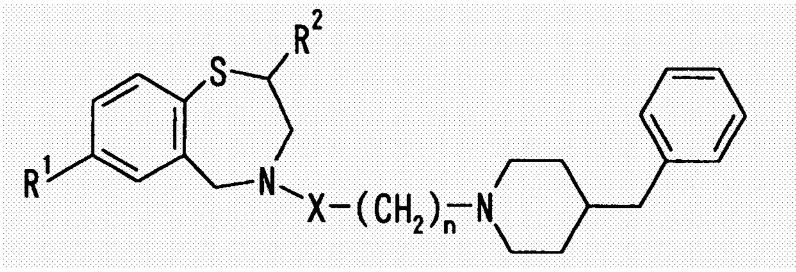

[I]

where $R^1$ represents a hydrogen atom or C1-C3 lower alkoxy group; $R^2$ represents a hydrogen atom, C1-C3 lower alkoxy group or phenyl group, wherein said phenyl group may be substituted with 1-3 substituents selected from the group consisting of a hydroxyl group and a C1-C3 lower alkoxy group, or a group represented by the following formula,

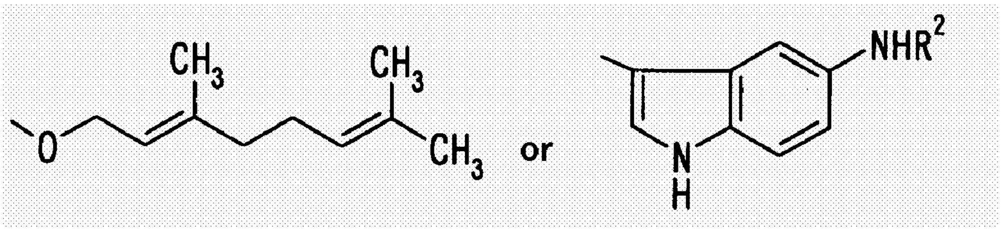

where $R^3$ represents a C1-C3 acyl group; X represents -CO- or $-CH_2-$; and n represents 1 or 2.

**16.** A therapeutic agent for acute pulmonary edema comprising a 1,4-benzothiazepine derivatives represented by the general formula [1] or a pharmaceutically acceptable salt thereof as defined in claim 14 or 15, wherein said 1,4-benzothiazepine derivatives is 4-[3-(4-benzylpiperidine-1-yl) propionyl]-7-methoxy-2,3,4,5 -tetrahydro- 1,4-benzothiazepine, or a pharmaceutically acceptable salt thereof.

**17.** An improving agent for vascular microcirculation comprising 1,4-benzothiazepine derivatives represented by the following general formula [I] or a pharmaceutically acceptable salt thereof as an active ingredient;

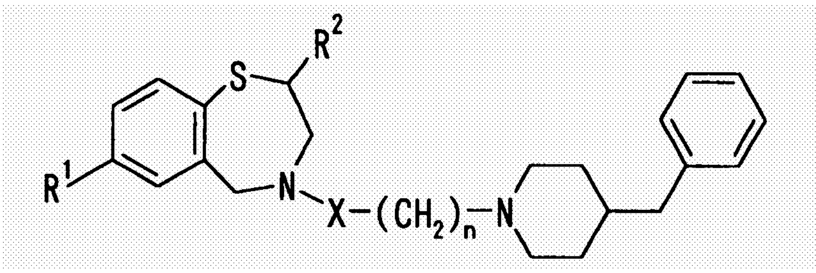

[I]

where $R^1$ represents a hydrogen atom or C1-C3 lower alkoxy group; $R^2$ represents a hydrogen atom, C1-C3 lower alkoxy group or phenyl group, wherein said phenyl group may be substituted with 1-3 substituents selected from the group consisting of a hydroxyl group and a C1-C3 lower alkoxy group, or a group represented by the following formula,

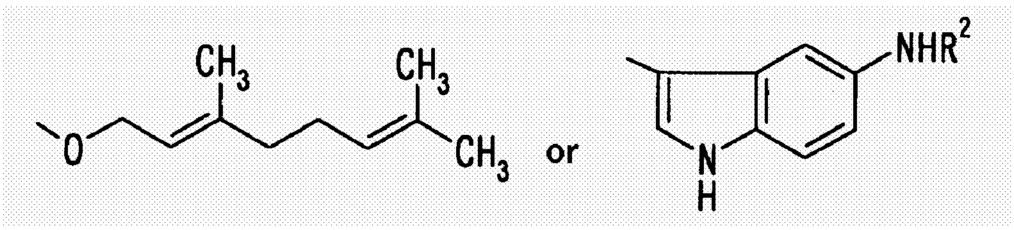

where $R^3$ represents a C1-C3 acyl group; X represents -CO- or $-CH_2-$; and n represents 1 or 2.

**18.** An improving agent for vascular microcirculation comprising 1,4-benzothiazepine derivatives represented by the following general formula [I] or a pharmaceutically acceptable salt thereof as an active ingredient and having an effect of enhancing binding strength to actin-tropomyosin complex of troponin I of a protein inhibiting muscle contraction in muscle;

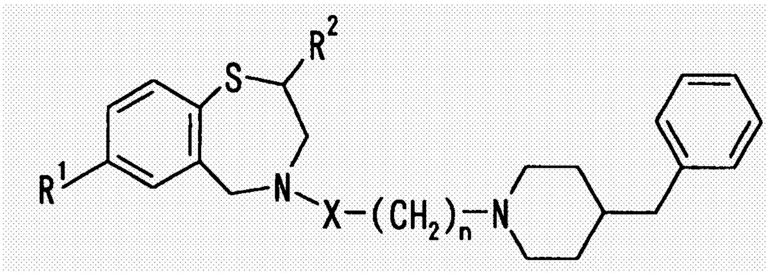

[I]

where $R^1$ represents a hydrogen atom or C1-C3 lower alkoxy group; $R^2$ represents a hydrogen atom, C1-C3 lower alkoxy group or phenyl group, wherein said phenyl group may be substituted with 1-3 substituents selected from the group consisting of a hydroxyl group and a C1-C3 lower alkoxy group, or a group represented by the following formula,

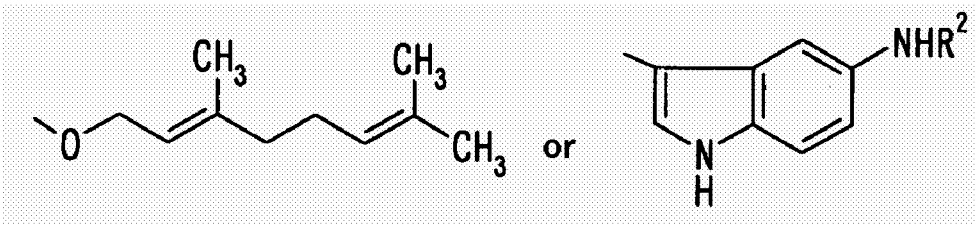

where $R^3$ represents a C1-C3 acyl group; X represents -CO- or $-CH_2-$; and n represents 1 or 2.

**19.** An improving agent for vascular microcirculation comprising a 1,4-benzothiazepine derivatives represented by the general formula [1] or a pharmaceutically acceptable salt thereof as defined in claim 17 or 18, wherein said 1,4-benzothiazepine derivatives is 4-[3-(4-benzylpiperidine-1-yl) propionyl]-7-methoxy-2,3,4,5 -tetrahydro- 1,4-benzothiazepine, or a pharmaceutically acceptable salt thereof.

**20.** A therapeutic agent for angina pectoris comprising 1,4-benzothiazepine derivatives represented by the following general formula [I] or a pharmaceutically acceptable salt thereof as an active ingredient;

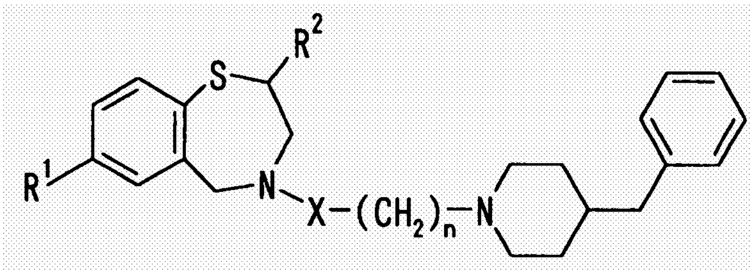

[I]

where $R^1$ represents a hydrogen atom or C1-C3 lower alkoxy group; $R^2$ represents a hydrogen atom, C1-C3 lower alkoxy group or phenyl group, wherein said phenyl group may be substituted with 1-3 substituents selected from the group consisting of a hydroxyl group and a C1-C3 lower alkoxy group, or a group represented by the following formula,

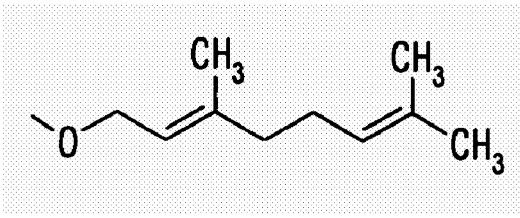 or 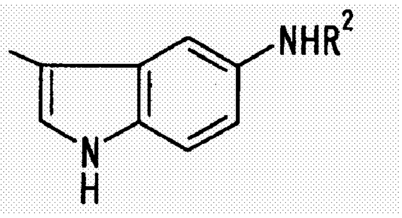

where $R^3$ represents a C1-C3 acyl group; X represents -CO- or $-CH_2-$; and n represents 1 or 2.

**21.** A therapeutic agent for angina pectoris comprising 1,4-benzothiazepine derivatives represented by the following general formula [I] or a pharmaceutically acceptable salt thereof as an active ingredient and having an effect of enhancing binding strength to actin-tropomyosin complex of troponin I of a protein inhibiting muscle contraction in muscle;

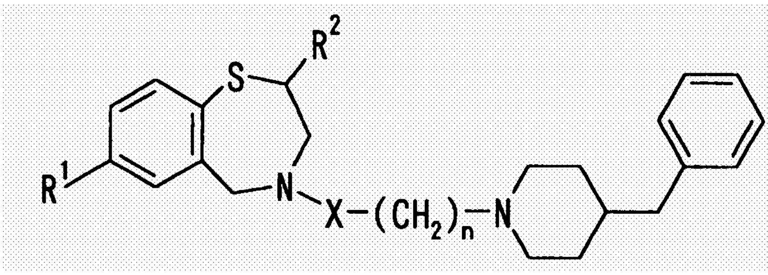

[I]

where $R^1$ represents a hydrogen atom or C1-C3 lower alkoxy group; $R^2$ represents a hydrogen atom, C1-C3 lower alkoxy group or phenyl group, wherein said phenyl group may be substituted with 1-3 substituents selected from the group consisting of a hydroxyl group and a C1-C3 lower alkoxy group, or a group represented by the following formula,

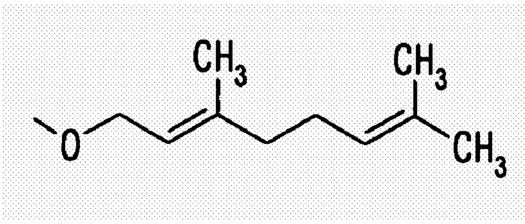 or 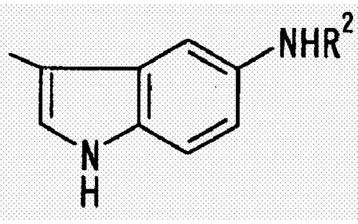

where $R^3$ represents a C1-C3 acyl group; X represents -CO- or $-CH_2-$; and n represents 1 or 2.

**22.** A therapeutic agent for angina pectoris comprising a 1,4-benzothiazepine derivatives represented by the general formula [1] or a pharmaceutically acceptable salt thereof as defined in claim 20 or 21, wherein said 1,4-benzothiazepine derivatives is 4-[3-(4-benzylpiperidine-1-yl) propionyl]-7-methoxy-2,3,4,5 -tetrahydro- 1,4-benzothiazepine, or a pharmaceutically acceptable salt thereof.

**23.** An improving agent for myocardiopathy comprising 1,4-benzothiazepine derivatives represented by the following general formula [I] or a pharmaceutically acceptable salt thereof as an active ingredient;

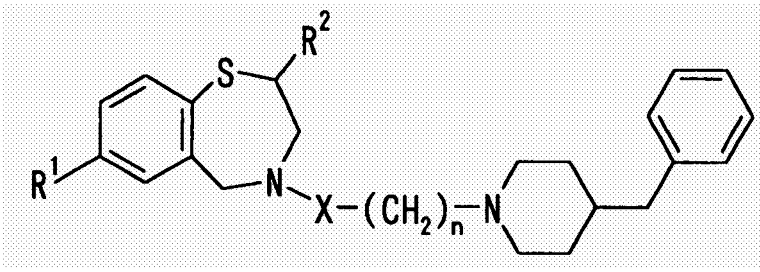

[I]

where $R^1$ represents a hydrogen atom or C1-C3 lower alkoxy group; $R^2$ represents a hydrogen atom, C1-C3 lower alkoxy group or phenyl group, wherein said phenyl group may be substituted with 1-3 substituents selected from the group consisting of a hydroxyl group and a C1-C3 lower alkoxy group, or a group represented by the following formula,

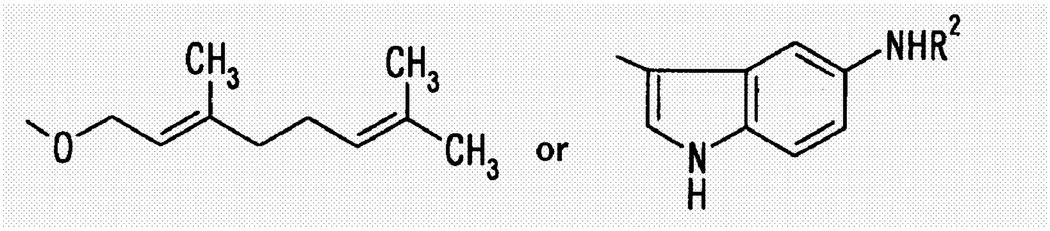

where $R^3$ represents a C1-C3 acyl group; X represents -CO- or $-CH_2-$; and n represents 1 or 2.

**24.** An improving agent for myocardiopathy comprising 1,4-benzothiazepine derivatives represented by the following general formula [I] or a pharmaceutically acceptable salt thereof as an active ingredient and having an effect of enhancing binding strength to actin-tropomyosin complex of troponin I of a protein inhibiting muscle contraction in muscle;

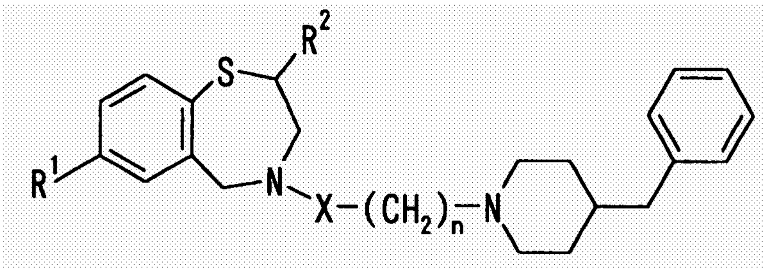

[I]

where $R^1$ represents a hydrogen atom or C1-C3 lower alkoxy group; $R^2$ represents a hydrogen atom, C1-C3 lower alkoxy group or phenyl group, wherein said phenyl group may be substituted with 1-3 substituents selected from the group consisting of a hydroxyl group and a C1-C3 lower alkoxy group, or a group represented by the following formula,

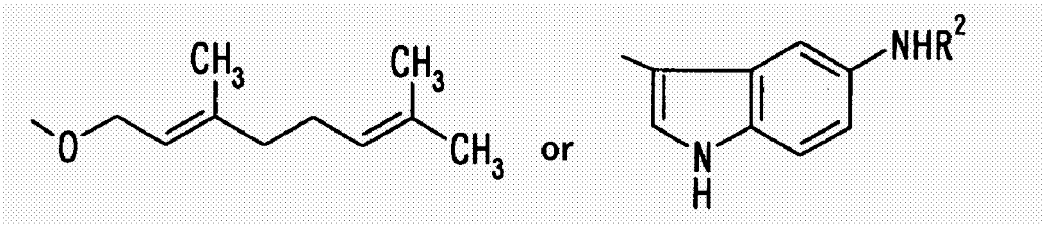

where $R^3$ represents a C1-C3 acyl group; X represents -CO- or $-CH_2-$; and n represents 1 or 2.

**25.** An improving agent for myocardiopathy comprising a 1,4-benzothiazepine derivatives represented by the general formula [1] or a pharmaceutically acceptable salt thereof as defined in claim 24 or 25, wherein said 1,4-benzothiazepine derivatives is 4-[3-(4-benzylpiperidine-1-yl) propionyl]-7-methoxy-2,3,4,5 -tetrahydro- 1,4-benzothiazepine, or a pharmaceutically acceptable salt thereof.

**26.** A therapeutic agent for hypertension comprising 1,4-benzothiazepine derivatives represented by the following general formula [I] or a pharmaceutically acceptable salt thereof as an active ingredient;

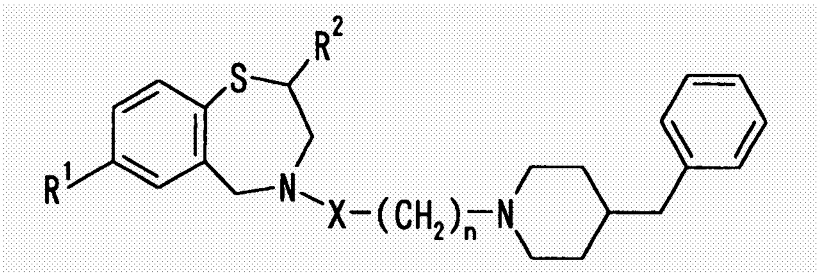

[I]

where $R^1$ represents a hydrogen atom or C1-C3 lower alkoxy group; $R^2$ represents a hydrogen atom, C1-C3 lower alkoxy group or phenyl group, wherein said phenyl group may be substituted with 1-3 substituents selected from the group consisting of a hydroxyl group and a C1-C3 lower alkoxy group, or a group represented by the following formula,

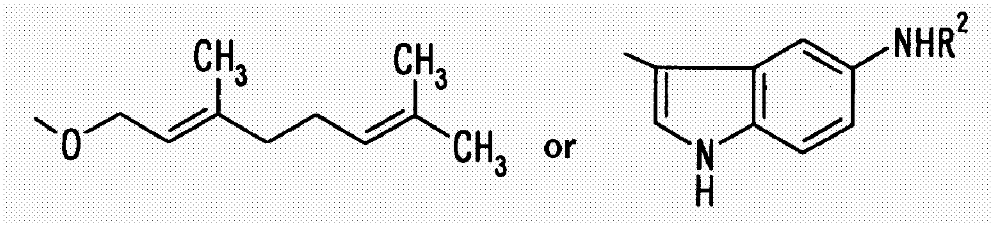

where $R^3$ represents a C1-C3 acyl group; X represents -CO- or $-CH_2-$; and n represents 1 or 2.

**27.** A therapeutic agent for hypertension comprising 1,4-benzothiazepine derivatives represented by the following general formula [I] or a pharmaceutically acceptable salt thereof as an active ingredient and having an effect of enhancing binding strength to actin-tropomyosin complex of troponin I of a protein inhibiting muscle contraction in muscle;

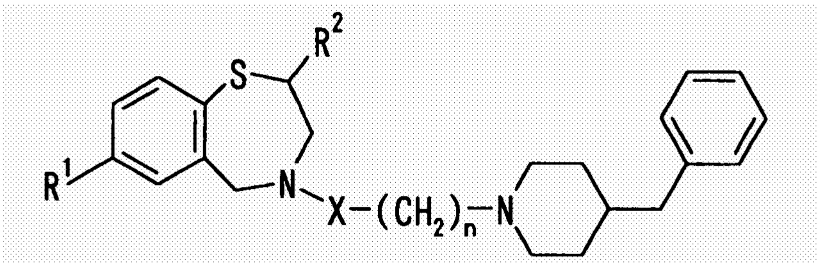

[I]

where $R^1$ represents a hydrogen atom or C1-C3 lower alkoxy group; $R^2$ represents a hydrogen atom, C1-C3 lower alkoxy group or phenyl group, wherein said phenyl group may be substituted with 1-3 substituents selected from the group consisting of a hydroxyl group and a C1-C3 lower alkoxy group, or a group represented by the following formula,

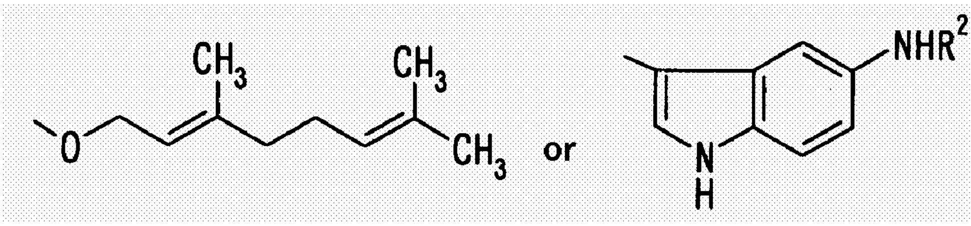

where $R^3$ represents a C1-C3 acyl group; X represents -CO- or $-CH_2-$; and n represents 1 or 2.

**28.** A therapeutic agent for hypertension comprising a 1,4-benzothiazepine derivatives represented by the general formula [1] or a pharmaceutically acceptable salt thereof as defined in claim 26 or 27, wherein said 1,4-benzothiazepine derivatives is 4-[3-(4-benzylpiperidine-1-yl) propionyl]-7-methoxy-2,3,4,5-tetrahydro-1,4-benzothiazepine, or a pharmaceutically acceptable salt thereof.

**29.** A therapeutic agent for ventricular tachycardia comprising 1,4-benzothiazepine derivatives represented by the following general formula [I] or a pharmaceutically acceptable salt thereof as an active ingredient;

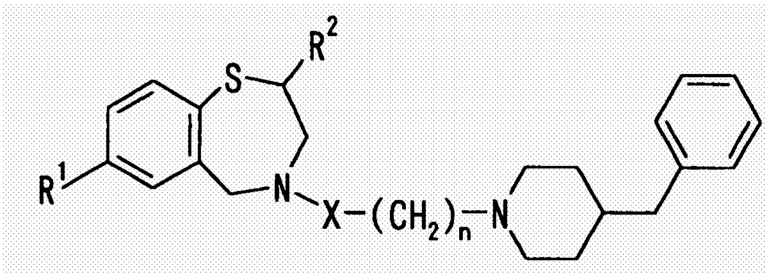

[I]

where $R^1$ represents a hydrogen atom or C1-C3 lower alkoxy group; $R^2$ represents a hydrogen atom, C1-C3 lower alkoxy group or phenyl group, wherein said phenyl group may be substituted with 1-3 substituents selected from the group consisting of a hydroxyl group and a C1-C3 lower alkoxy group, or a group represented by the following formula,

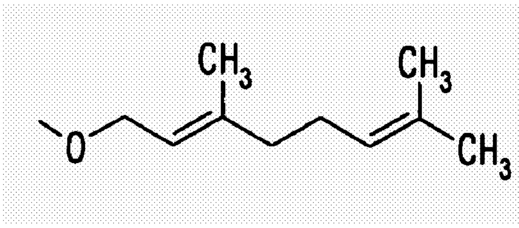

or

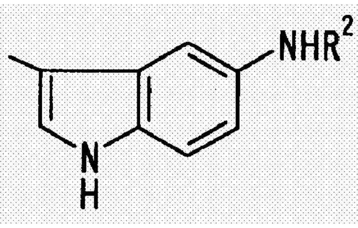

where $R^3$ represents a C1-C3 acyl group; X represents -CO- or $-CH_2-$; and n represents 1 or 2.

**30.** A therapeutic agent for ventricular tachycardia comprising 1,4-benzothiazepine derivatives represented by the following general formula [I] or a pharmaceutically acceptable salt thereof as an active ingredient and having an effect of enhancing binding strength to actin-tropomyosin complex of troponin I of a protein inhibiting muscle contraction in muscle;

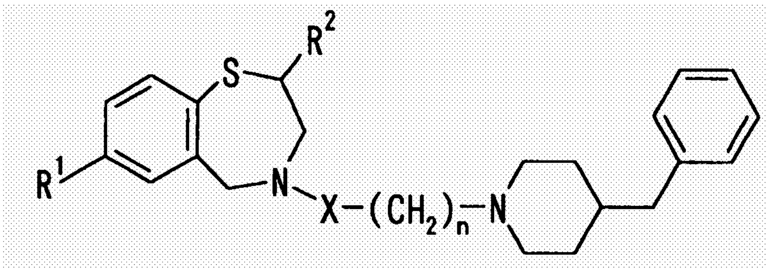

[I]

where $R^1$ represents a hydrogen atom or C1-C3 lower alkoxy group; $R^2$ represents a hydrogen atom, C1-C3 lower alkoxy group or phenyl group, wherein said phenyl group may be substituted with 1-3 substituents selected from the group consisting of a hydroxyl group and a C1-C3 lower alkoxy group, or a group represented by the following formula,

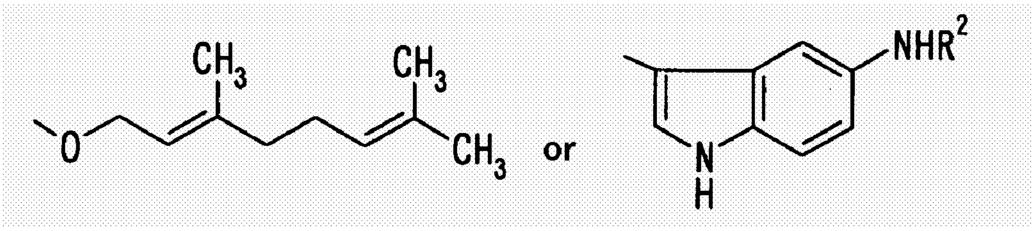

where $R^3$ represents a C1-C3 acyl group; X represents -CO- or $-CH_2-$; and n represents 1 or 2.

**31.** A therapeutic agent for ventricular tachycardia comprising a 1,4-benzothiazepine derivatives represented by the general formula [1] or a pharmaceutically acceptable salt thereof as defined in claim 29 or 30, wherein said 1,4-benzothiazepine derivatives is 4-[3-(4-benzylpiperidine-1-yl) propionyl]-7-methoxy-2,3,4,5 -tetrahydro- 1,4-benzothiazepine, or a pharmaceutically acceptable salt thereof.

**32.** A therapeutic agent for supraventricular tachycardia comprising 1,4-benzothiazepine derivatives represented by the following general formula [I] or a pharmaceutically acceptable salt thereof as an active ingredient;

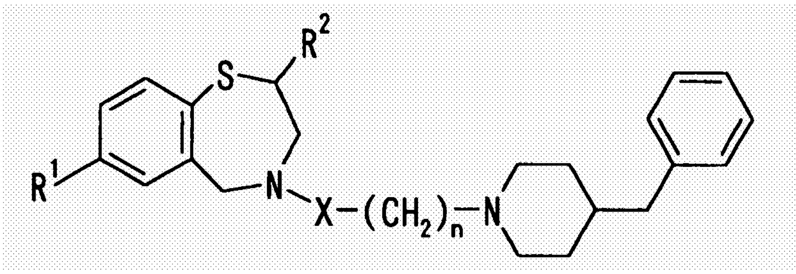

[I]

where $R^1$ represents a hydrogen atom or C1-C3 lower alkoxy group; $R^2$ represents a hydrogen atom, C1-C3 lower alkoxy group or phenyl group, wherein said phenyl group may be substituted with 1-3 substituents selected from the group consisting of a hydroxyl group and a C1-C3 lower alkoxy group, or a group represented by the following formula,

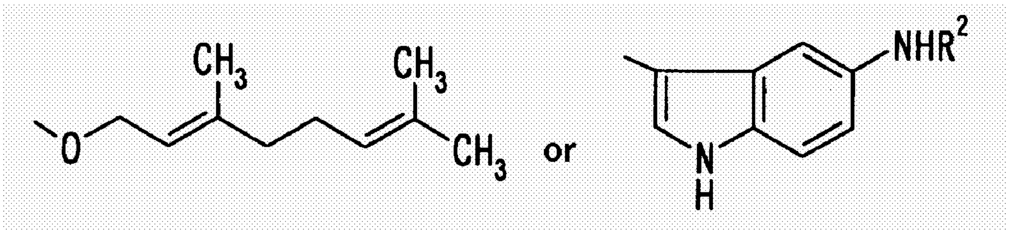

where $R^3$ represents a C1-C3 acyl group; X represents -CO- or $-CH_2-$; and n represents 1 or 2.

**33.** A therapeutic agent for supraventricular tachycardia comprising a 1,4-benzothiazepine derivatives represented by the following general formula [I] or a pharmaceutically acceptable salt thereof as an active ingredient and having an effect of enhancing binding strength to actin-tropomyosin complex of troponin I of a protein inhibiting muscle contraction in muscle;

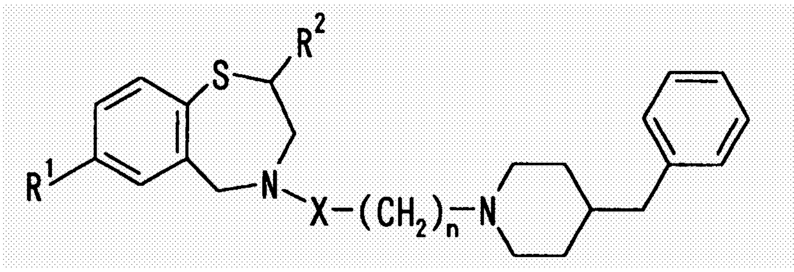

[I]

where $R^1$ represents a hydrogen atom or C1-C3 lower alkoxy group; $R^2$ represents a hydrogen atom, C1-C3 lower alkoxy group or phenyl group, wherein said phenyl group may be substituted with 1-3 substituents selected from the group consisting of a hydroxyl group and a C1-C3 lower alkoxy group, or a group represented by the following formula,

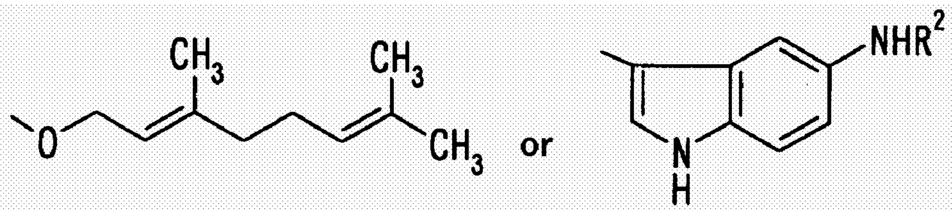

or

where $R^3$ represents a C1-C3 acyl group; X represents -CO- or $-CH_2-$; and n represents 1 or 2.

**34.** A therapeutic agent for supraventricular tachycardia comprising a 1,4-benzothiazepine derivatives represented by the general formula [1] or a pharmaceutically acceptable salt thereof as defined in claim 32 or 33, wherein said 1,4-benzothiazepine derivatives is 4-[3-(4-benzylpiperidine-1-yl) propionyl]-7-methoxy-2,3,4,5 -tetrahydro- 1,4-benzothiazepine, or a pharmaceutically acceptable salt thereof.

**35.** A therapeutic agent and prophylactic agent for torsades de pointes comprising 1,4-benzothiazepine derivatives represented by the following general formula [I] or a pharmaceutically acceptable salt thereof as an active ingredient;

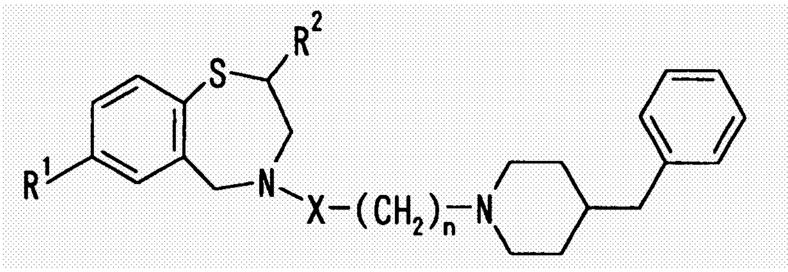

[I]

where $R^1$ represents a hydrogen atom or C1-C3 lower alkoxy group; $R^2$ represents a hydrogen atom, C1-C3 lower alkoxy group or phenyl group, wherein said phenyl group may be substituted with 1-3 substituents selected from the group consisting of a hydroxyl group and a C1-C3 lower alkoxy group, or a group represented by the following formula,

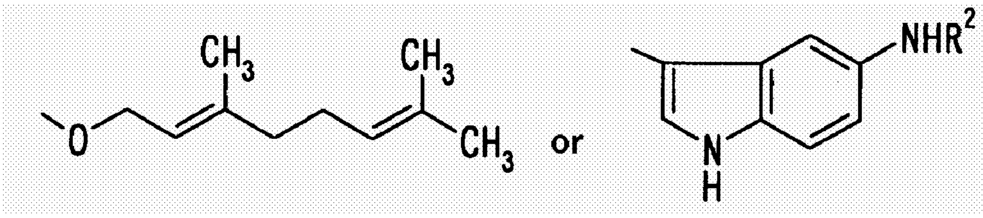

or

where $R^3$ represents a C1-C3 acyl group; X represents -CO- or $-CH_2-$; and n represents 1 or 2.

**36.** A therapeutic agent and prophylactic agent for torsades de pointes comprising a 1,4-benzothiazepine derivatives represented by the general formula [1] or a pharmaceutically acceptable salt thereof as defined in claim 35, wherein said 1,4-benzothiazepine derivatives is 4-[3-(4-benzylpiperidine-1-yl) propionyl]-7-methoxy-2,3,4,5 -tetrahydro- 1,4-benzothiazepine, or a pharmaceutically acceptable salt thereof.

FIG. 1

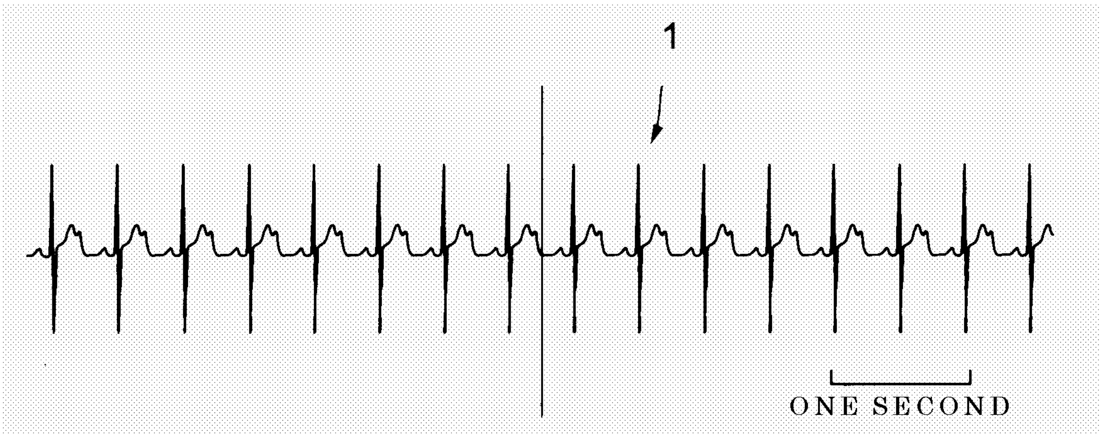


FIG. 2

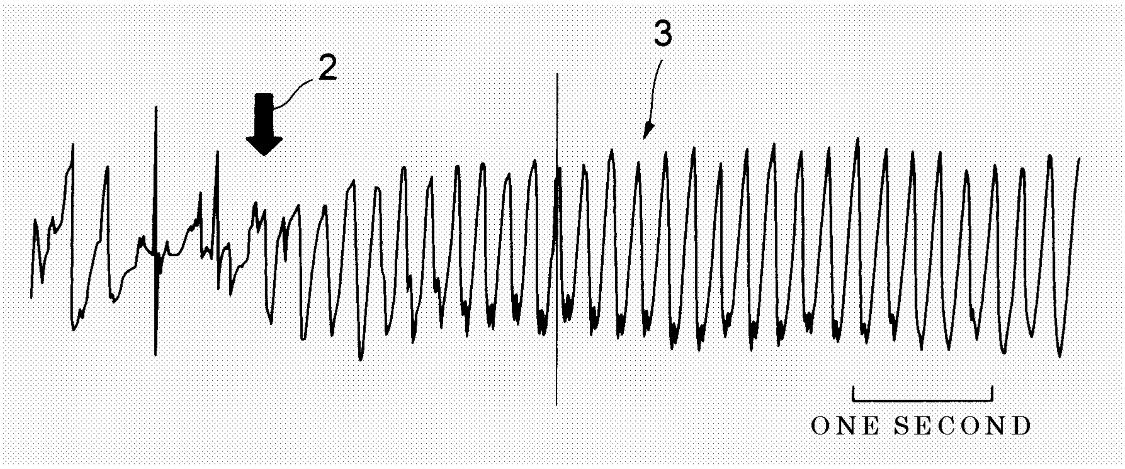


FIG. 3

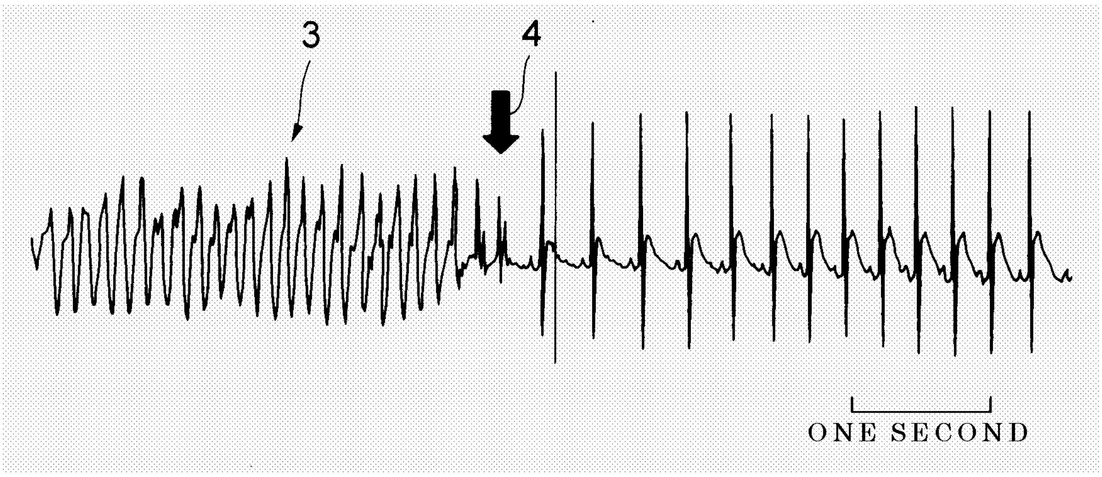

FIG. 4

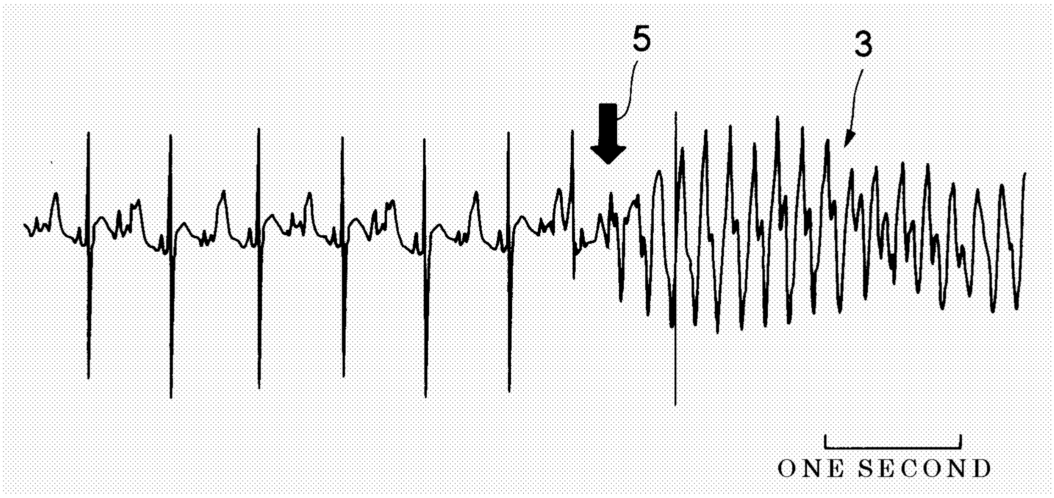


FIG. 5

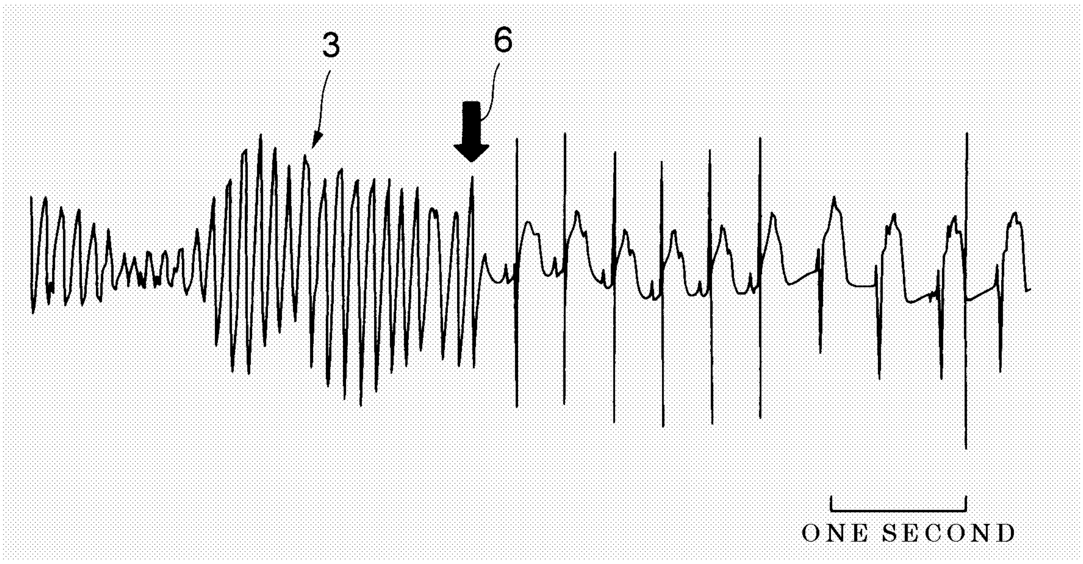

## INTERNATIONAL SEARCH REPORT

International application No.
PCT/JP2005/008563

A. CLASSIFICATION OF SUBJECT MATTER
Int.Cl[7] C07D417/06, A61K31/554, A61P9/00, 9/04, 9/10, 9/12, 11/00, 21/02, 43/00, C07D417/14

According to International Patent Classification (IPC) or to both national classification and IPC

B. FIELDS SEARCHED

Minimum documentation searched (classification system followed by classification symbols)
Int.Cl[7] C07D417/06, A61K31/554, A61P9/00, 9/04, 9/10, 9/12, 11/00, 21/02, 43/00, C07D417/14

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2005 |
| Kokai Jitsuyo Shinan Koho | 1971-2005 | Toroku Jitsuyo Shinan Koho | 1994-2005 |

Electronic data base consulted during the international search (name of data base and, where practicable, search terms used)
CA(STN), MEDLINE(STN), REGISTRY(STN)

C. DOCUMENTS CONSIDERED TO BE RELEVANT

| Category* | Citation of document, with indication, where appropriate, of the relevant passages | Relevant to claim No. |
|---|---|---|
| X<br><br>A | SHIMAMOTO, Ken et al., Effects of a novel 1,4-benzothiazepine derivative, K201, on cytosolic Ca2+ and contraction in isolated smooth mustle of rat aorta, Tokyo Joshi Ika Daigaku Zasshi, 2004, Vol.74, No.1, pages 39 to 46 | 1-7,14-19, 26-28<br>8-10 |
| X<br><br>A | JP 2003-95977 A (Masafumi YANO), 03 April, 2003 (03.04.03), Full text (Family: none) | 1-7,11-16, 20-25<br>8-10 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 28 July, 2005 (28.07.05) | 23 August, 2005 (23.08.05) |
| Name and mailing address of the ISA/<br>Japanese Patent Office<br>Facsimile No. | Authorized officer<br><br>Telephone No. |

Form PCT/ISA/210 (second sheet) (January 2004)

**INTERNATIONAL SEARCH REPORT**

International application No.
PCT/JP2005/008563

C (Continuation). DOCUMENTS CONSIDERED TO BE RELEVANT

| Category* | Citation of document, with indication, where appropriate, of the relevant passages | Relevant to claim No. |
|---|---|---|
| X | JP 4-230681 A (Kirin Brewery Co., Ltd.), 19 August, 1992 (19.08.92), Full text & WO 92/12148 A1 & CA 2098495 A & AU 9191074 A & ZA 9110166 A & EP 565721 A1 & CN 1063491 A & US 5416066 A | 11-16 |
| X | US 2003/0220312 A1 (Joseph R. Schuh), 27 November, 2003 (27.11.03), Full text; particularly, Claims & US 2002/132001 A1 & US 2002/042405 A1 & US 2003/055027 A1 | 11-16 |
| X | JP 2004-0048780 A1 (Andrew R. Marks), 11 March, 2004 (11.03.04), Full text; particularly, Claims & US 6489125 A & WO 2005/002518 A2 | 11-16,32-36 |
| X | JP 2000-247889 A (Japan Tobacco Inc.), 12 September, 2000 (12.09.00), Full text & WO 2000/038688 A1 & CA 2357038 A & EP 1147772 A1 & US 6506745 B1 | 11-16,26-31 |

Form PCT/ISA/210 (continuation of second sheet) (January 2004)

INTERNATIONAL SEARCH REPORT

International application No.
PCT/JP2005/008563

· With respect to symbol in the general formula [I]

The general formula which involves an indole ring and is shown as one of the choices for $R^2$ in the general formula [I] also includes "$R^2$." The explanation on this general formula does not include "$R^2$" and is made on "$R^3$," which is not included in that formula. Consequently, the "$R^2$" in the general formula involving an indole ring is considered to be a clerical error for "$R^3$."

· With respect to claims 14-16

Claims 14-16 include the term "a therapeutic agent for acute pulmonary edema." However, acute pulmonary edema caused by the aggravation of cardiac failure is the only acute pulmonary edema described in the description. It is considered that there is no disclosure of a therapeutic agent for acute pulmonary edema attributable to any other cause. Therefore, with respect to claims 14-16, a search was made only for a therapeutic agent for acute pulmonary edema attributable to cardiac failure.

· Claim 28 includes the term "the therapeutic agent for hypertension of claim 27 or 28." Namely, claim 28 is cited in itself.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 2003095977 A
- JP 2703408 B

### Non-patent literature cited in the description

- *J. Biochem.,* 2002, vol. 131, 739-743